# EUROPEAN PATENT APPLICATION

(11) **EP 4 668 778 A1**
(43) Date of publication of application: **24.12.2025**
(21) Application number: 24773768.7
(22) Date of filing: 11.01.2024
(51) Int. Cl.: H04R 25/00

(54) **HEARING LOSS SIMULATION SYSTEM, EARPHONE, AND SERVER**

(30) Priority: 22.03.2023 CN 202310312346
(71) Applicant: Huawei Technologies Co., Ltd., Shenzhen, Guangdong 518129 (CN)
(72) Inventor: QU, Kaiyang, Shenzhen, Guangdong 518129 (CN); PEI, Shengsheng, Shenzhen, Guangdong 518129 (CN); ZHOU, Lei, Shenzhen, Guangdong 518129 (CN); QIN, Peng, Shenzhen, Guangdong 518129 (CN)
(74) Representative: Körber, Martin Hans
(86) International application number: PCT/CN2024/071727
(87) International publication number: WO 2024/193198

(57) **Abstract**

Embodiments of this application provide a hearing loss simulation system, a headset, and a server. The hearing loss simulation system includes a headset and a terminal device, and the headset is worn by a first user. The terminal device is configured to: receive an information input operation, and obtain user information of a second user that is correspondingly input by the information input operation; and receive a listening test operation, and send listening test data to the headset in response to the listening test operation, where the listening test data includes the user information or hearing loss feature information, and the hearing loss feature information is determined based on the user information. The headset is configured to: obtain first audio data; perform hearing loss simulation on the first audio data based on the hearing loss feature information, to obtain second audio data; and play the second audio data. In this way, the first user can use the hearing loss simulation system to experience an effect of audio heard by the second user.

## Description

This application claims priority to Chinese Patent Application No. 202310312346.4, filed with the China National Intellectual Property Administration on March 22, 2023, and entitled "HEARING LOSS SIMULATION SYSTEM, HEADSET, AND SERVER", which is incorporated herein by reference in its entirety.

### TECHNICAL FIELD

Embodiments of this application relate to the audio processing field, and in particular, to a hearing loss simulation system, a headset, and a server.

### BACKGROUND

Aging, physiological lesions, mechanical damage to the ears (or head), or the like can cause hearing loss. A user with hearing loss cannot hear or hear clearly the voice of another person, music, radio, or other sound like a normal person (that is, a user without hearing loss), which causes many inconveniences to the user with hearing loss in daily life, work, and other processes.

A user with normal hearing (that is, without hearing loss) usually cannot understand auditory experience of the user with hearing loss, and cannot comprehend difficulties in the life and work of the user with hearing loss. Further, in a process of communicating with the user with hearing loss, ineffective communication usually occurs, a requirement of the user with hearing loss is ignored, and even a misunderstanding or conflict occurs.

### SUMMARY

In view of this, this application provides a hearing loss simulation system, a headset, and a server. The hearing loss simulation system can simulate audio heard by any user. In this way, a first user can experience, based on the hearing loss simulation system, an effect of audio heard by a second user.

According to a first aspect, an embodiment of this application provides a hearing loss simulation system. The hearing loss simulation system includes a headset and a terminal device, and the headset is worn by a first user, where
the terminal device is configured to: receive an information input operation, and obtain user information of a second user that is correspondingly input by the information input operation; and receive a listening test operation, and send listening test data to the headset in response to the listening test operation, where the listening test data includes the user information or hearing loss feature information, and the hearing loss feature information is determined based on the user information; and
the headset is configured to: obtain first audio data; perform hearing loss simulation on the first audio data based on the hearing loss feature information, to obtain second audio data; and play the second audio data.

In this way, after the first user inputs the user information of the second user on the terminal device and performs the listening test operation, the first user can hear, in the headset, the audio data heard by the second user, and experience an effect of audio heard by the second user.

In a possible manner, the first user may be a user without hearing loss, and the second user may be a user with hearing loss. In this way, the user without hearing loss can experience, based on the hearing loss simulation system, an effect of audio heard by the user with hearing loss, so that the first user can be more patient when communicating with the second user, and in a process of communicating with the second user, speak louder, repeat words more frequently, and reduce a speaking speed.

In a possible manner, the first user may be a user with a lower degree of hearing loss, and the second user may be a user with a higher degree of hearing loss. In this way, the user with the lower degree of hearing loss can experience, based on the hearing loss simulation system, an effect of audio heard by the user with the higher degree of hearing loss, so that the first user can be more patient when communicating with the second user, and in a process of communicating with the second user, speak louder, repeat words more frequently, and reduce a speaking speed.

In a family scenario, both the first user and the second user may be family members, the second user is a grandfather, and the first user is a grandson. After experiencing an effect of audio heard by the grandfather, and perceiving feelings of the grandfather and inconveniences caused by hearing loss in life, the grandson can be prompted to buy a hearing aid for the grandfather; and the grandson can be more patient when communicating with the grandfather, and in a process of communicating with the grandfather, speak louder, repeat words more frequently, and reduce a speaking speed.

In another scenario, for example, in a working scenario, the first user is an employee of a human resources department, and the second user is an employee of a logistics department. After experiencing an effect of audio data heard by the employee of the logistics department, the employee of the human resources department can perceive feelings of the employee of the logistics department and inconveniences caused by hearing loss in life and work. In this way, the employee of the human resources department can be prompted to be more patient when communicating with the employee of the logistics department, and in a process of communicating with the employee of the logistics department, speak louder, repeat words more frequently, and reduce a speaking speed.

It should be understood that the second user may alternatively be a user without hearing loss. This is not limited in this application. This application is described by using an example in which the first user is a user without hearing loss and the second user is a user with hearing loss.

For example, the user information may include but is not limited to: an ear shape photo, an age, a gender, hearing loss duration, hearing aid wearing duration, intelligibility test data, an audiogram, and the like. This is not limited in this application.

For example, the hearing loss feature information may include but is not limited to ear-shaped features (such as an auricle length, an auricle width, an auricle depth, and an ear type), hearing loss frequency band information, an intelligibility score, an intelligibility confusion matrix, age-related decline information (such as an age, a gender, hearing loss duration, and hearing aid wearing duration), and the like. This is not limited in this application.

For example, ear-shaped features such as an auricle length, an auricle width, an auricle depth, and an ear type may be extracted by analyzing an ear shape photo. There may be a plurality of ear types, for example, cup-shaped ears, cauliflower ears, and protruding ears. This is not limited in this application. It should be understood that the ear-shaped features are not limited in this application.

For example, a degree of hearing loss (unit: dB) in each sub-band (that is, within different frequency ranges) may be extracted by analyzing an audiogram, and is used as hearing loss frequency band information.

For example, an intelligibility score and an intelligibility confusion matrix may be determined by analyzing intelligibility test data. A higher intelligibility score of audio data indicates that the audio data is easier to understand.

It should be noted that this application does not limit whether the terminal device determines the hearing loss feature information based on the user information, or the headset determines the hearing loss feature information based on the user information.

For example, when the listening test operation is a selection operation for a preset audio scenario option, the listening test data further includes the first audio data, and the first audio data is preset audio data corresponding to the preset audio option corresponding to the selection operation; or when the listening test operation is a selection operation for a real-time audio option, the listening test data further includes an audio collection start indication, and the audio collection start indication indicates the headset to collect audio.

For example, a preset audio scenario may be a scenario in which corresponding preset audio data can be obtained in advance, for example, a dish washing scenario or a vegetable market scenario, and does not include a real-time audio scenario. In this way, the first user can experience an effect of audio heard by the second user when the second user is in the preset audio scenario, and can experience an effect of audio heard by the second user in real time when the second user is in a current scenario.

According to the first aspect, the terminal device is further configured to display first evaluation information of the first audio data and second evaluation information of the second audio data. In this way, a hearing gap between a normal person and a person with hearing loss can be quantified, which better helps the second user feel inconveniences in life of the first user; and further, the first user can be more patient when communicating with the second user, and in the process of communicating with the second user, speak louder, repeat words more frequently, and reduce the speaking speed; or there is greater motivation to buy a hearing aid subsequently.

For example, the first evaluation information may be information obtained by performing objective evaluation (which may also be referred to as objective assessment) on the first audio data. For example, the first evaluation information may include but is not limited to a first intelligibility score and a first sound quality score.

For example, the second evaluation information may be information obtained by performing objective evaluation on the second audio data, for example, may include but is not limited to a second intelligibility score and a second sound quality score.

For example, a higher intelligibility score of the audio data indicates that the audio data is easier to understand.

For example, a higher sound quality score of the audio data indicates better sound quality of the audio data.

For example, the first intelligibility score is greater than the second intelligibility score, and the first sound quality score is greater than the second sound quality score.

According to any one of the first aspect or the foregoing implementations of the first aspect, the terminal device is further configured to display text information corresponding to the first audio data, and annotate a target word in the text information based on the second audio data, where an intelligibility score of the target word is less than an intelligibility threshold, and/or the target word is a word whose sound quality score is less than a sound quality threshold. In this way, the first user can conveniently learn audio of words that the second user cannot hear or cannot clearly hear.

For example, the intelligibility threshold and the sound quality threshold may be set based on a requirement. This is not limited in this application.

It should be understood that the target word may be annotated in a plurality of manners, for example, in a manner of changing a color, adding an underline, or using boldface. This is not limited in this application.

For example, the sound quality may be quality of sound or quality of sound. Specifically, the sound quality is a comprehensive representation in a plurality of dimensions such as clarity, naturalness, timbre, latency, and jitter. A manner of evaluating the sound quality score may include subjective evaluation (for example, a mean opinion score (Mean Opinion Score, MOS)) and objective evaluation (for example, perceptual evaluation of speech quality (Perceptual evaluation of speech quality, PESQ)).

For example, intelligibility may be a percentage of voice signals that are transmitted by using a sound transmission system and that can be understood by a listener (for example, if the listener listens to 100 words and correctly understands 50 words, an intelligibility score of the words is 50%), or may be speech clarity (speech intelligibility).

According to any one of the first aspect or the foregoing implementations of the first aspect, the headset is further configured to play the first audio data. In this way, the first user can conveniently compare audio data heard by a normal person with audio data heard by a user with hearing loss, to better experience a difference between the audio data heard by the normal person and the audio data heard by the user with hearing loss; the first user can be more patient when communicating with the second user, and in the process of communicating with the second user, speak louder, repeat words more frequently, and reduce the speaking speed; or there is greater motivation to buy a hearing aid subsequently.

For example, the headset may play the first audio data before or after playing the second audio data.

According to any one of the first aspect or the foregoing implementations of the first aspect, before receiving the listening test operation, the terminal device is further configured to receive an experience time setting operation, and obtain experience time information that is correspondingly set by the experience time setting operation, where the listening test data further includes the experience time information, and the hearing loss feature information is determined based on the user information and the experience time information. In this way, the first user can experience an effect of audio heard by the second user at an age corresponding to the experience time information.

For example, the experience time information may be in units of years. For example, the experience time information may be "0", "-5", "+5", and "+10", where "0" represents present, "-5" represents 5 years ago, "+5" represents 5 years later, and "+10" represents 10 years later.

In this case, an age in the age-related decline information may be a sum of the age in the user information and the experience time information.

According to any one of the first aspect or the foregoing implementations of the first aspect, before receiving the listening test operation, the terminal device is further configured to receive a hearing aid type selection operation, and obtain hearing aid type information corresponding to the hearing aid type selection operation, where the listening test data further includes the hearing aid type information; and the headset is further configured to:
perform hearing aid optimization processing on the first audio data by using a hearing aid algorithm corresponding to the hearing aid type information, to obtain third audio data; and
perform hearing loss simulation processing on the third audio data based on the hearing loss feature information, to obtain the second audio data.

In this way, the first user may select different hearing aid types from the terminal device, to experience effects of audio heard by the second user when the second user wears different types of hearing aids; and with reference to subjective perception and objective evaluation information (including the first evaluation information and the second evaluation information), select a hearing aid type with an optimal effect, to configure a most appropriate hearing aid type for the first user.

In a possible manner, the headset may be used as a hearing aid. After determining the hearing aid type with the optimal effect, the first user may select the corresponding hearing aid type on the terminal device. In this way, in a subsequent process in which the second user uses the headset as a hearing aid, the headset may invoke a hearing aid algorithm corresponding to a hearing aid type option to perform hearing aid optimization processing, and send the third audio data obtained through the hearing aid optimization processing to a speaker for playing (in this case, no hearing loss simulation is required).

In a possible manner, after determining the hearing aid type with the optimal effect, the first user may buy a hearing aid based on the hearing aid type with the optimal effect.

In this case, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

According to any one of the first aspect or the foregoing implementations of the first aspect, before receiving the listening test operation, the terminal device is further configured to receive a first parameter setting operation, and obtain a first hearing aid algorithm parameter corresponding to the first parameter setting operation, where the listening test data further includes the first hearing aid algorithm parameter; and the headset is further configured to: perform hearing aid optimization processing on the first audio data based on the first hearing aid algorithm parameter, to obtain fourth audio data; and perform hearing loss simulation processing on the fourth audio data based on the hearing loss feature information, to obtain the second audio data.

In this way, by configuring different first hearing aid algorithm parameters in the terminal device, the first user can experience audio data heard by the second user after the second user wears hearing aids with different hearing aid algorithm parameters; and select an optimal first hearing aid algorithm parameter with reference to subjective perception and objective evaluation information (for example, including the first evaluation information and the second evaluation information), to configure an appropriate first hearing aid algorithm parameter for the first user.

In a possible manner, the headset may be used as a hearing aid. After determining the first hearing aid algorithm parameter with an optimal effect, the first user may perform a configuration on the terminal device based on the optimal first hearing aid algorithm parameter. In this way, in a subsequent process in which the second user uses the headset as a hearing aid, the hearing aid algorithm of the headset may perform hearing aid optimization processing by using the first hearing aid algorithm parameter with the optimal effect, and send the fourth audio data obtained through the hearing aid optimization processing to the speaker for playing (in this case, no hearing loss simulation is required).

In a possible manner, after determining the first hearing aid algorithm parameter with the optimal effect, the first user may buy a hearing aid based on the first hearing aid algorithm parameter with the optimal effect.

The first hearing aid algorithm parameter is a part of the second hearing aid algorithm parameter (the second hearing aid algorithm parameter is all parameters required for calculation by the hearing aid algorithm).

In this case, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

According to any one of the first aspect or the foregoing implementations of the first aspect, before receiving the listening test operation, the terminal device is further configured to receive a second parameter setting operation, and obtain an optimization parameter corresponding to the second parameter setting operation, where the listening test data further includes the optimization parameter; and the headset is further configured to: search for a second hearing aid algorithm parameter based on the optimization parameter; perform hearing aid optimization processing on the first audio data based on the second hearing aid algorithm parameter, to obtain fifth audio data; and perform hearing loss simulation processing on the fifth audio data based on the hearing loss feature information, to obtain the second audio data.

In this way, the first user may configure different second hearing aid algorithm parameters by setting different optimization parameters in the terminal device, to experience audio data heard by the second user after the second user wears hearing aids with different hearing aid algorithm parameters; and select an optimal optimization parameter with reference to subjective perception and objective evaluation information (including the first evaluation information and the second evaluation information), to configure an appropriate second hearing aid algorithm parameter for the first user.

In a possible manner, the headset may be used as a hearing aid. After determining the optimization parameter with an optimal effect, the first user may perform a configuration on the terminal device based on the optimal optimization parameter. In this way, in a subsequent process in which the second user uses the headset as a hearing aid, the hearing aid algorithm of the headset may perform hearing aid optimization processing by using a second hearing aid algorithm parameter with an optimal effect, and send the fifth audio data obtained through the hearing aid optimization processing to the speaker for playing (in this case, no hearing loss simulation is required).

In a possible manner, after determining the optimization parameter with the optimal effect, the first user may view, from the terminal device, the second hearing aid algorithm parameter with the optimal effect; and then may buy a hearing aid based on the second hearing aid algorithm parameter with the optimal effect.

In this case, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

According to any one of the first aspect or the foregoing implementations of the first aspect, the headset is further configured to: perform hearing loss simulation on the first audio data based on the hearing loss feature information, to obtain sixth audio data; and play the sixth audio data; and the terminal device is further configured to display third evaluation information of the sixth audio data. In this way, the first user may compare an effect of audio that is heard by the second user by using no hearing aid with an effect of audio that is heard by the second user by using a hearing aid, to understand an effect of the hearing aid on the second user, so that there is greater motivation to buy a hearing aid subsequently.

For example, the third evaluation information may be information obtained by performing objective evaluation on the third audio data. For example, the third evaluation information may include but is not limited to a third intelligibility score and/or a third sound quality score.

For example, the headset may sequentially play the first audio data, the second audio data, and the sixth audio data.

According to any one of the first aspect or the foregoing implementations of the first aspect, the headset is specifically configured to: determine a hearing loss simulation parameter based on the hearing loss feature information, where the hearing loss simulation parameter includes a hair cell filter coefficient; and perform hair cell filtering processing on the first audio data based on the hair cell filter coefficient, to obtain the second audio data. A cochlea (which may include hair cells (including inner hair cells (inner hear cells, IHCs) and outer hair cells (outer hear cell, OHCs))) that can modulate audio is one of key elements that enable a user to clearly hear audio content. Therefore, the second audio data obtained by simulating audio modulation by the IHCs and the OHCs and performing hair cell filtering processing on the first audio data is closer to the audio data actually heard by the second user.

According to any one of the first aspect or the foregoing implementations of the first aspect, the hearing loss simulation parameter further includes an ear-shaped filter coefficient and a recruitment coefficient; and the headset is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data. Compared with the conventional technology, in this application, an actual auditory pathway such as inner/outer hair cells and cochlear reflexes of an ear is considered, and impact of audio intelligibility, a degree of hearing loss, an age, and the like on physiological parameters in the auditory pathway is considered. Therefore, the hearing loss simulation in this application better conforms to a physiological process of an auditory periphery, and can simulate audio data closer to audio data heard by a user with hearing loss.

According to a second aspect, an embodiment of this application provides a hearing loss simulation system. The hearing loss simulation system includes a headset, a terminal device, and a server, and the headset is worn by a first user, where
the terminal device is configured to: receive an information input operation, and obtain user information of a second user that is correspondingly input by the information input operation; and receive a listening test operation, and send listening test data to the server in response to the listening test operation, where the listening test data includes the user information or hearing loss feature information, and the hearing loss feature information is determined based on the user information;
the server is configured to: obtain first audio data; perform hearing loss simulation processing on the first audio data based on the hearing loss feature information, to obtain second audio data; and send the second audio data to the terminal device;
the terminal device is further configured to send the second audio data to the headset; and
the headset is configured to play the second audio data.

According to the second aspect, the terminal device is further configured to display first evaluation information of the first audio data and second evaluation information of the second audio data.

According to any one of the second aspect or the foregoing implementations of the second aspect, the terminal device is further configured to display text information corresponding to the first audio data, and annotate a target word in the text information based on the second audio data, where
an intelligibility score of the target word is less than an intelligibility threshold, and/or the target word is a word whose sound quality score is less than a sound quality threshold.

According to any one of the second aspect or the foregoing implementations of the second aspect, the headset is further configured to play the first audio data.

According to any one of the second aspect or the foregoing implementations of the second aspect, before receiving the listening test operation, the terminal device is further configured to receive an experience time setting operation, and obtain experience time information that is correspondingly set by the experience time setting operation, where the listening test data further includes the experience time information, and the hearing loss feature information is determined based on the user information and the experience time information.

According to any one of the second aspect or the foregoing implementations of the second aspect, before receiving the listening test operation, the terminal device is further configured to receive a hearing aid type selection operation, and obtain hearing aid type information corresponding to the hearing aid type selection operation, where the listening test data further includes the hearing aid type information; and the server is further configured to: perform hearing aid optimization processing on the first audio data by using a hearing aid algorithm corresponding to the hearing aid type information, to obtain third audio data; and perform hearing loss simulation processing on the third audio data based on the hearing loss feature information, to obtain the second audio data.

According to any one of the second aspect or the foregoing implementations of the second aspect, before receiving the listening test operation, the terminal device is further configured to receive a first parameter setting operation, and obtain a first hearing aid algorithm parameter corresponding to the first parameter setting operation, where the listening test data further includes the first hearing aid algorithm parameter; and the server is further configured to: perform hearing aid optimization processing on the first audio data based on the first hearing aid algorithm parameter, to obtain fourth audio data; and perform hearing loss simulation processing on the fourth audio data based on the hearing loss feature information, to obtain the second audio data.

According to any one of the second aspect or the foregoing implementations of the second aspect, before receiving the listening test operation, the terminal device is further configured to receive a second parameter setting operation, and obtain an optimization parameter corresponding to the second parameter setting operation, where the listening test data further includes the optimization parameter; and the server is further configured to:
search for a second hearing aid algorithm parameter based on the optimization parameter;
perform hearing aid optimization processing on the first audio data based on the second hearing aid algorithm parameter, to obtain fifth audio data; and perform hearing loss simulation processing on the fifth audio data based on the hearing loss feature information, to obtain the second audio data.

According to any one of the second aspect or the foregoing implementations of the second aspect, the server is further configured to: perform hearing loss simulation on the first audio data based on the hearing loss feature information, to obtain sixth audio data; and send the sixth audio data to the terminal device; the terminal device is further configured to send the sixth audio data to the headset; the headset is further configured to play the sixth audio data; and the terminal device is further configured to display third evaluation information of the sixth audio data.

According to any one of the second aspect or the foregoing implementations of the second aspect, the server is specifically configured to: determine a hearing loss simulation parameter based on the hearing loss feature information, where the hearing loss simulation parameter includes a hair cell filter coefficient; and perform hair cell filtering processing on the first audio data based on the hair cell filter coefficient, to obtain the second audio data.

According to any one of the second aspect or the foregoing implementations of the second aspect, the hearing loss feature information further includes an ear-shaped filter coefficient and a recruitment coefficient; and the server is specifically configured to:
perform ear-shaped migration processing on the first audio data based on the ear-shaped filter coefficient, to obtain first intermediate audio data; perform cochlear conversion processing on the first intermediate audio data, to obtain second intermediate audio data;
perform hair cell filtering processing on the second intermediate audio data based on the hair cell filter coefficient, to obtain third intermediate audio data; perform spectral smearing processing on the third intermediate audio data, to obtain fourth intermediate audio data;
perform loudness recruitment processing on the fourth intermediate audio data based on the recruitment coefficient, to obtain fifth intermediate audio data; and perform cochlear reverse conversion processing on the fifth intermediate audio data, to obtain the second audio data.

The second aspect and any one of the implementations of the second aspect respectively correspond to the first aspect and any one of the implementations of the first aspect. For technical effects corresponding to the second aspect and any one of the implementations of the second aspect, refer to technical effects corresponding to the first aspect and any one of the implementations of the first aspect. Details are not described herein again.

According to a third aspect, an embodiment of this application provides a hearing loss simulation system. The hearing loss simulation system includes a headset and a terminal device, and the headset is worn by a first user, where

the terminal device is configured to: receive an information input operation, and obtain user information of a second user that is correspondingly input by the information input operation; receive a listening test operation, obtain first audio data in response to the listening test operation, and generate hearing loss feature information based on the user information; perform hearing loss simulation on the first audio data based on the hearing loss feature information, to obtain second audio data; and send the second audio data to the headset; and the headset is configured to play the second audio data.

According to the third aspect, the terminal device is further configured to display first evaluation information of the first audio data and second evaluation information of the second audio data.

According to any one of the third aspect or the foregoing implementations of the third aspect, the terminal device is further configured to display text information corresponding to the first audio data, and annotate a target word in the text information based on the second audio data, where an intelligibility score of the target word is less than an intelligibility threshold, and/or the target word is a word whose sound quality score is less than a sound quality threshold.

According to any one of the third aspect or the foregoing implementations of the third aspect, the headset is further configured to play the first audio data.

According to any one of the third aspect or the foregoing implementations of the third aspect, before receiving the listening test operation, the terminal device is further configured to receive an experience time setting operation, and obtain experience time information that is correspondingly set by the experience time setting operation, where the listening test data further includes the experience time information, and the hearing loss feature information is determined based on the user information and the experience time information.

According to any one of the third aspect or the foregoing implementations of the third aspect, before receiving the listening test operation, the terminal device is further configured to receive a hearing aid type selection operation, and obtain hearing aid type information corresponding to the hearing aid type selection operation, where the listening test data further includes the hearing aid type information; and the terminal device is further configured to: perform hearing aid optimization processing on the first audio data by using a hearing aid algorithm corresponding to the hearing aid type information, to obtain third audio data; and perform hearing loss simulation processing on the third audio data based on the hearing loss feature information, to obtain the second audio data.

According to any one of the third aspect or the foregoing implementations of the third aspect, before receiving the listening test operation, the terminal device is further configured to receive a first parameter setting operation, and obtain a first hearing aid algorithm parameter corresponding to the first parameter setting operation, where the listening test data further includes the first hearing aid algorithm parameter; and the terminal device is further configured to: perform hearing aid optimization processing on the first audio data based on the first hearing aid algorithm parameter, to obtain fourth audio data; and perform hearing loss simulation processing on the fourth audio data based on the hearing loss feature information, to obtain the second audio data.

According to any one of the third aspect or the foregoing implementations of the third aspect, before receiving the listening test operation, the terminal device is further configured to receive a second parameter setting operation, and obtain an optimization parameter corresponding to the second parameter setting operation, where the listening test data further includes the optimization parameter; and the terminal device is further configured to: search for a second hearing aid algorithm parameter based on the optimization parameter; perform hearing aid optimization processing on the first audio data based on the second hearing aid algorithm parameter, to obtain fifth audio data; and perform hearing loss simulation processing on the fifth audio data based on the hearing loss feature information, to obtain the second audio data.

According to any one of the third aspect or the foregoing implementations of the third aspect, the terminal device is further configured to: perform hearing loss simulation on the first audio data based on the hearing loss feature information, to obtain sixth audio data; and send the sixth audio data to the headset; the headset is configured to play the sixth audio data; and the terminal device is further configured to display third evaluation information of the sixth audio data.

According to any one of the third aspect or the foregoing implementations of the third aspect, the terminal device is specifically configured to: determine a hearing loss simulation parameter based on the hearing loss feature information, where the hearing loss simulation parameter includes a hair cell filter coefficient; and perform hair cell filtering processing on the first audio data based on the hair cell filter coefficient, to obtain the second audio data.

According to any one of the third aspect or the foregoing implementations of the third aspect, the hearing loss feature information further includes an ear-shaped filter coefficient and a recruitment coefficient; and the terminal device is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

The third aspect and any one of the implementations of the third aspect respectively correspond to the first aspect and any one of the implementations of the first aspect. For technical effects corresponding to the third aspect and any one of the implementations of the third aspect, refer to technical effects corresponding to the first aspect and any one of the implementations of the first aspect. Details are not described herein again.

According to a fourth aspect, this application further provides a headset. The headset is worn by a first user, and the headset is configured to:
receive listening test data, where the listening test data includes user information of a second user or hearing loss feature information, and the hearing loss feature information is determined based on the user information;
obtain first audio data;
determine a hearing loss simulation parameter based on the hearing loss feature information, where the hearing loss simulation parameter includes a hair cell filter coefficient;
perform hair cell filtering processing on the first audio data based on the hair cell filter coefficient, to obtain second audio data; and
play the second audio data.

According to the fourth aspect, the hearing loss simulation parameter further includes an ear-shaped filter coefficient and a recruitment coefficient; and the headset is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

According to any one of the fourth aspect or the foregoing implementations of the fourth aspect, the headset is configured to: when the listening test data further includes the first audio data, extract the first audio data from the listening test data; or when the listening test data further includes an audio collection start indication, start an audio collection module to collect the first audio data.

According to any one of the fourth aspect or the foregoing implementations of the fourth aspect, before performing hair cell filtering processing on the first audio data based on the hair cell filter coefficient, to obtain the second audio data, the headset is further configured to:
when the listening test data further includes hearing aid type information, perform hearing aid optimization processing on the first audio data by using a hearing aid algorithm corresponding to the hearing aid type information;
when the listening test data further includes a first hearing aid algorithm parameter, perform hearing aid optimization processing on the first audio data based on the first hearing aid algorithm parameter; or
when the listening test data further includes an optimization parameter, search for a second hearing aid algorithm parameter based on the optimization parameter; and perform hearing aid optimization processing on the first audio data based on the second hearing aid algorithm parameter.

According to any one of the fourth aspect or the foregoing implementations of the fourth aspect, the hearing loss feature information includes at least one of the following: an ear-shaped feature, hearing loss frequency band information, an intelligibility score, an intelligibility confusion matrix, or age-related decline information.

The fourth aspect and any one of the implementations of the fourth aspect respectively correspond to the first aspect and any one of the implementations of the first aspect. For technical effects corresponding to the fourth aspect and any one of the implementations of the fourth aspect, refer to technical effects corresponding to the first aspect and any one of the implementations of the first aspect. Details are not described herein again.

According to a fifth aspect, an embodiment of this application provides a server. The server is configured to:
receive listening test data, where the listening test data includes user information of a second user or hearing loss feature information, and the hearing loss feature information is determined based on the user information;
obtain first audio data;
determine a hearing loss simulation parameter based on the hearing loss feature information, where the hearing loss simulation parameter includes a hair cell filter coefficient;
perform hair cell filtering processing on the first audio data based on the hair cell filter coefficient, to obtain second audio data; and
send the second audio data to a headset, so that the headset plays the second audio data, where the headset is worn by a first user.

According to the fifth aspect, the hearing loss simulation parameter further includes an ear-shaped filter coefficient and a recruitment coefficient; and the server is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

According to any one of the fifth aspect or the foregoing implementations of the fifth aspect, before performing hair cell filtering processing on the first audio data based on the hair cell filter coefficient, to obtain the second audio data, the server is further configured to:
when the listening test data further includes hearing aid type information, perform hearing aid optimization processing on the first audio data by using a hearing aid algorithm corresponding to the hearing aid type information;
when the listening test data further includes a first hearing aid algorithm parameter, perform hearing aid optimization processing on the first audio data based on the first hearing aid algorithm parameter; or
when the listening test data further includes an optimization parameter, search for a second hearing aid algorithm parameter based on the optimization parameter; and perform hearing aid optimization processing on the first audio data based on the second hearing aid algorithm parameter.

According to any one of the fifth aspect or the foregoing implementations of the fifth aspect, the hearing loss feature information includes at least one of the following: an ear-shaped feature, hearing loss frequency band information, an intelligibility score, an intelligibility confusion matrix, or age-related decline information.

The fifth aspect and any one of the implementations of the fifth aspect respectively correspond to the first aspect and any one of the implementations of the first aspect. For technical effects corresponding to the fifth aspect and any one of the implementations of the fifth aspect, refer to technical effects corresponding to the first aspect and any one of the implementations of the first aspect. Details are not described herein again.

According to a sixth aspect, this application provides a terminal device. The terminal device is configured to:
receive an information input operation, and obtain user information of a second user that is correspondingly input by the information input operation; and
receive a listening test operation, and send listening test data to a headset in response to the listening test operation, where the listening test data includes the user information or hearing loss feature information, the hearing loss feature information is determined based on the user information, the hearing loss feature information is used by the headset to perform hearing loss simulation on first audio data to obtain second audio data, the second audio data is played by the headset, and the headset is worn by a first user.

According to the sixth aspect, the terminal device is further configured to display first evaluation information of the first audio data and second evaluation information of the second audio data.

According to any one of the sixth aspect or the foregoing implementations of the sixth aspect, the terminal device is further configured to display text information corresponding to the first audio data, and annotate a target word in the text information based on the second audio data, where an intelligibility score of the target word is less than an intelligibility threshold, and/or the target word is a word whose sound quality score is less than a sound quality threshold.

According to any one of the sixth aspect or the foregoing implementations of the sixth aspect, before receiving the listening test operation, the terminal device is further configured to receive an experience time setting operation, and obtain experience time information that is correspondingly set by the experience time setting operation, where the hearing loss feature information is determined based on the user information and the experience time information.

According to any one of the sixth aspect or the foregoing implementations of the sixth aspect, when the listening test operation is a selection operation for a preset audio option, the listening test data further includes the first audio data, and the first audio data is preset audio data corresponding to the preset audio option corresponding to the selection operation; or when the listening test operation is a selection operation for a real-time audio option, the listening test data further includes an audio collection start indication, and the audio collection start indication indicates the headset to collect audio.

According to any one of the sixth aspect or the foregoing implementations of the sixth aspect, before receiving the listening test operation, the terminal device is further configured to receive a hearing aid type selection operation, and obtain hearing aid type information corresponding to the hearing aid type selection operation, where the listening test data further includes the hearing aid type information, the hearing aid type information is used by the headset to determine a hearing aid algorithm, and the hearing aid algorithm is used by the headset to perform hearing aid optimization processing on the first audio data.

According to any one of the sixth aspect or the foregoing implementations of the sixth aspect, before receiving the listening test operation, the terminal device is further configured to receive a first parameter setting operation, and obtain a first hearing aid algorithm parameter corresponding to the first parameter setting operation, where the listening test data further includes the first hearing aid algorithm parameter, and the first hearing aid algorithm parameter is used by the headset to perform hearing aid optimization processing on the first audio data.

According to any one of the sixth aspect or the foregoing implementations of the sixth aspect, before receiving the listening test operation, the terminal device is further configured to receive a second parameter setting operation, and obtain an optimization parameter corresponding to the second parameter setting operation, where the listening test data further includes the optimization parameter, the optimization parameter is used by the headset to search for a second hearing aid algorithm parameter, and the second hearing aid algorithm parameter is used by the headset to perform hearing aid optimization processing on the first audio data.

According to any one of the sixth aspect or the foregoing implementations of the sixth aspect, the terminal device is further configured to display third evaluation information of sixth audio data, where the sixth audio data is obtained by the headset by performing hearing loss simulation on the first audio data based on the hearing loss feature information.

According to any one of the sixth aspect or the foregoing implementations of the sixth aspect, the terminal device is further configured to: receive a first identity selection operation before receiving the first parameter setting operation, where the first parameter setting operation is a setting operation for a first hearing aid algorithm parameter corresponding to a first identity; or receive a second identity selection operation before receiving the first parameter setting operation, where the first parameter setting operation is a setting operation for a first hearing aid algorithm parameter corresponding to a second identity.

For example, the first hearing aid algorithm parameter corresponding to the first identity is different from the first hearing aid algorithm parameter corresponding to the second identity. In this way, users with different identities can set different first hearing aid algorithm parameters. The first hearing aid algorithm parameter corresponding to the first identity may include but is not limited to noise reduction strength, gain, EQ, and the like. This is not limited in this application. The first hearing aid algorithm parameter corresponding to the second identity may include but is not limited to noise reduction strength, beam strength, a degree of howling suppression, and the like. This is not limited in this application.

For example, the first identity is a non-professional physician, and the first identity is a professional physician. In this way, when the first user is a professional physician, a hearing aid algorithm parameter with a better hearing aid effect can be configured for the second user.

According to any one of the sixth aspect or the foregoing implementations of the sixth aspect, the user information includes at least one of the following: an ear shape photo, hearing test data, or age information.

For example, when the first user is a physician, user information of the first user, such as a medical license, may be uploaded.

For example, historical user information (such as a historical audiogram and historical intelligibility test data) of the second user, a historical first hearing aid algorithm parameter, and the like may also be uploaded.

The sixth aspect and any one of the implementations of the sixth aspect respectively correspond to the first aspect and any one of the implementations of the first aspect. For technical effects corresponding to the sixth aspect and any one of the implementations of the sixth aspect, refer to technical effects corresponding to the first aspect and any one of the implementations of the first aspect. Details are not described herein again.

According to a seventh aspect, this application provides a terminal device. The terminal device is configured to:
receive an information input operation, and obtain user information of a second user that is correspondingly input by the information input operation;
receive a listening test operation, obtain first audio data in response to the listening test operation, and generate hearing loss feature information based on the user information;
perform hearing loss simulation on the first audio data based on the hearing loss feature information, to obtain second audio data; and
send the second audio data to a headset, so that the headset plays the second audio data, where the headset is worn by a first user.

It should be understood that the terminal device in the seventh aspect may perform the steps performed by the terminal device in the third aspect. Details are not described herein again.

In addition, according to the seventh aspect, the terminal device is further configured to: receive a first identity selection operation before receiving a first parameter setting operation, where the first parameter setting operation is a setting operation for a first hearing aid algorithm parameter corresponding to a first identity; or receive a second identity selection operation before receiving a first parameter setting operation, where the first parameter setting operation is a setting operation for a first hearing aid algorithm parameter corresponding to a second identity.

The seventh aspect and any one of implementations of the seventh aspect respectively correspond to the first aspect and any one of the implementations of the first aspect. For technical effects corresponding to the seventh aspect and any one of the implementations of the seventh aspect, refer to technical effects corresponding to the first aspect and any one of the implementations of the first aspect. Details are not described herein again.

It should be noted that evaluation information (which may include first evaluation information, second evaluation information, and third evaluation information) may be calculated by the terminal device, a server, the headset, or another device. This is not limited in this application. For example, when the terminal device calculates the evaluation information, the terminal device may display the evaluation information, or may transmit the evaluation information to another terminal device to display the evaluation information. When the headset/server calculates the evaluation information, the headset/server may transmit the evaluation information to the terminal device or another terminal device to display the evaluation information. In addition, after calculating the evaluation information to obtain the evaluation information through calculation, the terminal device, the server, the headset, or the another device may store the evaluation information for subsequent viewing and use. In other words, a type of an electronic device for calculating the evaluation information is not limited in this application, and a manner of using the evaluation information is not limited either.

It should be further noted that the three processing operations of hearing loss simulation, hearing aid optimization, and searching for a second hearing aid algorithm parameter may be decoupled. In other words, two or more processing operations of hearing loss simulation, hearing aid optimization, and searching for the second hearing aid algorithm parameter may be performed by a same device, or may be performed by different devices. This is not limited in this application.

According to an eighth aspect, this application provides a hearing loss simulation apparatus. The hearing loss simulation apparatus is configured to: obtain first audio data; and perform hair cell filtering processing on the first audio data, to obtain second audio data.

According to the eighth aspect, the hearing loss simulation apparatus is specifically configured to: perform ear-shaped migration processing on the first audio data, to obtain first intermediate audio data; perform cochlear conversion processing on the first intermediate audio data, to obtain second intermediate audio data; perform hair cell filtering processing on the second intermediate audio data based on a hair cell filter coefficient, to obtain third intermediate audio data; perform spectral smearing processing on the third intermediate audio data, to obtain fourth intermediate audio data; perform loudness recruitment processing on the fourth intermediate audio data, to obtain fifth intermediate audio data; and perform cochlear reverse conversion processing on the fifth intermediate audio data, to obtain the second audio data.

The eighth aspect and any one of implementations of the eighth aspect respectively correspond to the first aspect and any one of the implementations of the first aspect. For technical effects corresponding to the eighth aspect and any one of the implementations of the eighth aspect, refer to technical effects corresponding to the first aspect and any one of the implementations of the first aspect. Details are not described herein again.

According to a ninth aspect, an embodiment of this application provides a headset, including a memory and a processor. The memory is coupled to the processor. The memory stores program instructions, and when the program instructions are executed by the processor, the headset is enabled to perform the steps in any one of the fourth aspect or possible implementations of the fourth aspect.

The ninth aspect and any one of implementations of the ninth aspect respectively correspond to the first aspect and any one of the implementations of the first aspect. For technical effects corresponding to the ninth aspect and any one of the implementations of the ninth aspect, refer to technical effects corresponding to the first aspect and any one of the implementations of the first aspect. Details are not described herein again.

According to a tenth aspect, an embodiment of this application provides a server, including a memory and a processor. The memory is coupled to the processor. The memory stores program instructions, and when the program instructions are executed by the processor, the server is enabled to perform the steps in any one of the fifth aspect or possible implementations of the fifth aspect.

The tenth aspect and any one of implementations of the tenth aspect respectively correspond to the first aspect and any one of the implementations of the first aspect. For technical effects corresponding to the tenth aspect and any one of the implementations of the tenth aspect, refer to technical effects corresponding to the first aspect and any one of the implementations of the first aspect. Details are not described herein again.

According to an eleventh aspect, an embodiment of this application provides a terminal device, including a memory and a processor. The memory is coupled to the processor. The memory stores program instructions, and when the program instructions are executed by the processor, the terminal device is enabled to perform the steps in any one of the sixth aspect (or the seventh aspect) or possible implementations of the sixth aspect (or the seventh aspect).

The eleventh aspect and any one of implementations of the eleventh aspect respectively correspond to the first aspect and any one of the implementations of the first aspect. For technical effects corresponding to the eleventh aspect and any one of the implementations of the eleventh aspect, refer to technical effects corresponding to the first aspect and any one of the implementations of the first aspect. Details are not described herein again.

According to a twelfth aspect, an embodiment of this application provides a chip, including one or more interface circuits and one or more processors. The interface circuit is configured to receive a signal from a memory of a headset, and send the signal to the processor, where the signal includes computer instructions stored in the memory. When the processor executes the computer instructions, the headset is enabled to perform the steps in any one of the fourth aspect or possible implementations of the fourth aspect.

The twelfth aspect and any one of implementations of the twelfth aspect respectively correspond to the first aspect and any one of the implementations of the first aspect. For technical effects corresponding to the twelfth aspect and any one of the implementations of the twelfth aspect, refer to technical effects corresponding to the first aspect and any one of the implementations of the first aspect. Details are not described herein again.

According to a thirteenth aspect, an embodiment of this application provides a chip, including one or more interface circuits and one or more processors. The interface circuit is configured to receive a signal from a memory of a server, and send the signal to the processor, where the signal includes computer instructions stored in the memory. When the processor executes the computer instructions, the server is enabled to perform the steps in any one of the fifth aspect or possible implementations of the fifth aspect.

The thirteenth aspect and any one of implementations of the thirteenth aspect respectively correspond to the first aspect and any one of the implementations of the first aspect. For technical effects corresponding to the thirteenth aspect and any one of the implementations of the thirteenth aspect, refer to technical effects corresponding to the first aspect and any one of the implementations of the first aspect. Details are not described herein again.

According to a fourteenth aspect, an embodiment of this application provides a chip, including one or more interface circuits and one or more processors. The interface circuit is configured to receive a signal from a memory of a terminal device, and send the signal to the processor, where the signal includes computer instructions stored in the memory. When the processor executes the computer instructions, the terminal device is enabled to perform the steps in any one of the sixth aspect (or the seventh aspect) or possible implementations of the sixth aspect (or the seventh aspect).

The fourteenth aspect and any one of implementations of the fourteenth aspect respectively correspond to the first aspect and any one of the implementations of the first aspect. For technical effects corresponding to the fourteenth aspect and any one of the implementations of the fourteenth aspect, refer to technical effects corresponding to the first aspect and any one of the implementations of the first aspect. Details are not described herein again.

According to a fifteenth aspect, an embodiment of this application provides a computer-readable storage medium. The computer-readable storage medium stores a computer program. When the computer program is run on a computer or a processor, the computer or the processor is enabled to perform the steps in any one of the fourth aspect (or the fifth aspect, or the sixth aspect, or the seventh aspect) or possible implementations of the fourth aspect (or the fifth aspect, or the sixth aspect, or the seventh aspect).

The fifteenth aspect and any one of implementations of the fifteenth aspect respectively correspond to the first aspect and any one of the implementations of the first aspect. For technical effects corresponding to the fifteenth aspect and any one of the implementations of the fifteenth aspect, refer to technical effects corresponding to the first aspect and any one of the implementations of the first aspect. Details are not described herein again.

According to a sixteenth aspect, an embodiment of this application provides a computer program product. The computer program product includes computer instructions. When the computer instructions are executed by a computer or a processor, the computer or the processor is enabled to perform the steps in any one of the fourth aspect (or the fifth aspect, or the sixth aspect, or the seventh aspect) or possible implementations of the fourth aspect (or the fifth aspect, or the sixth aspect, or the seventh aspect).

The sixteenth aspect and any one of implementations of the sixteenth aspect respectively correspond to the first aspect and any one of the implementations of the first aspect. For technical effects corresponding to the sixteenth aspect and any one of the implementations of the sixteenth aspect, refer to technical effects corresponding to the first aspect and any one of the implementations of the first aspect. Details are not described herein again.

### BRIEF DESCRIPTION OF DRAWINGS

FIG. 1a is a diagram of an example of an application scenario;
FIG. 1b is a diagram of an example of a hearing loss simulation system;
FIG. 1c is a diagram of an example of a hearing loss simulation system;
FIG. 1d is a diagram of an example of a hearing loss simulation system;
FIG. 2 is a diagram of an example of a hearing loss experience process;
FIG. 3a(1) to FIG. 3a(4) are diagrams of examples of mobile phone interfaces;
FIG. 3b is a diagram of an example of a hearing loss simulation system;
FIG. 3c is a diagram of an example of an intelligibility confusion matrix;
FIG. 3d(1) and FIG. 3d(2) are diagrams of examples of mobile phone interfaces;
FIG. 4a(1) and FIG. 4a(2) are diagrams of examples of mobile phone interfaces;
FIG. 4b is a diagram of an example of a hearing loss simulation system;
FIG. 5a(1) to FIG. 5a(3) are diagrams of examples of mobile phone interfaces;
FIG. 5b(1) to FIG. 5b(4) are diagrams of examples of mobile phone interfaces;
FIG. 6a(1) and FIG. 6a(2) are diagrams of examples of mobile phone interfaces;
FIG. 6b is a diagram of an example of a hearing loss simulation system;
FIG. 7 is a diagram of an example of a mobile phone interface;
FIG. 8 is a diagram of an example of a hearing loss experience process;
FIG. 9 is a diagram of an example of a hearing loss experience process; and
FIG. 10 is a diagram of an example of a structure of an apparatus.

### DESCRIPTION OF EMBODIMENTS

The following clearly and completely describes the technical solutions in embodiments of this application with reference to the accompanying drawings in embodiments of this application. It is clear that the described embodiments are some but not all of embodiments of this application. All other embodiments obtained by a person of ordinary skill in the art based on embodiments of this application without creative efforts shall fall within the protection scope of this application.

The term "and/or" in this specification describes only an association relationship between associated objects and represents that three relationships may exist. For example, A and/or B may represent the following three cases: Only A exists, both A and B exist, and only B exists.

In the specification and claims in embodiments of this application, the terms "first", "second", and the like are intended to distinguish between different objects, but are not used to describe a specific order of the objects. For example, a first target object, a second target object, and the like are used to distinguish between different target objects, but are not used to describe a specific order of the target objects.

In embodiments of this application, the term "example", "for example", or the like represents an example, an illustration, or a description. Any embodiment or design scheme described as "example" or "for example" in embodiments of this application should not be construed as being more preferred or advantageous than another embodiment or design scheme. To be precise, the terms such as "example" or "for example" are intended to present a relative concept in a specific manner.

In the descriptions of embodiments of this application, unless otherwise specified, "a plurality of" means two or more. For example, a plurality of processing units mean two or more processing units, and a plurality of systems mean two or more systems.

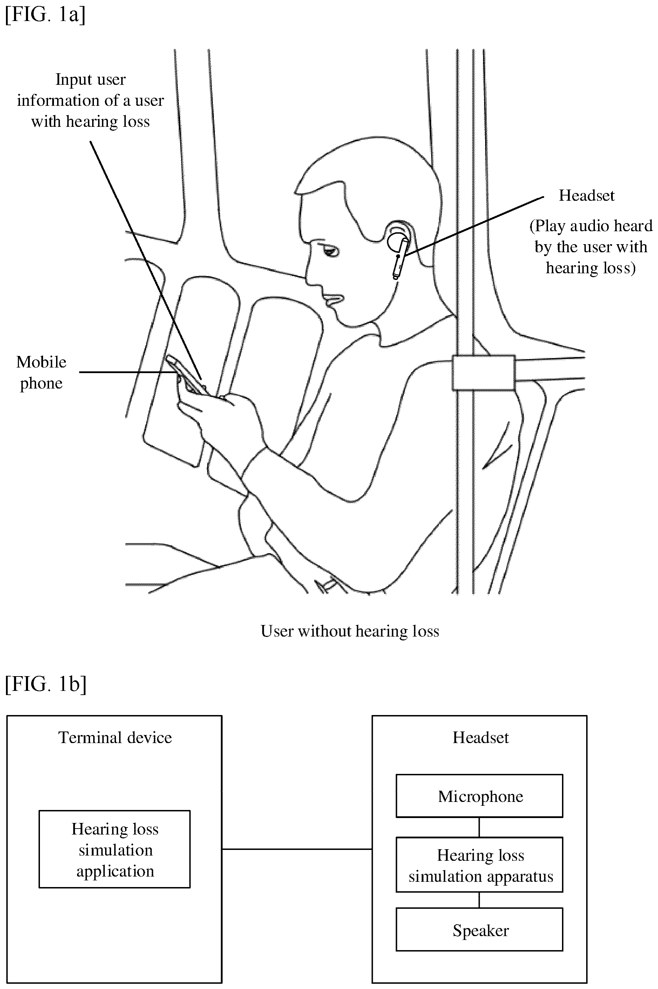

FIG. 1a is a diagram of an example of an application scenario. FIG. 1a shows a scenario in which a user without hearing loss experiences an effect of audio heard by a user with hearing loss. It should be understood that this application may be further applied to a scenario in which a user with a lower degree of hearing loss experiences an effect of audio heard by a user with a higher degree of hearing loss. In addition, this application may be further applied to a scenario in which a user without hearing loss experiences an effect of audio heard by another user without hearing loss. This is not limited in this application.

With reference to FIG. 1a, for example, when a user without hearing loss needs to experience an effect of audio heard by a user with hearing loss, the user without hearing loss may connect a headset to a mobile phone, and wear the headset. Then in a hearing loss simulation application of the mobile phone, the user without hearing loss may input user information of the user with hearing loss, and then may hear, in the headset, audio heard by the user with hearing loss.

It should be understood that the headset in FIG. 1a is merely an example in this application. The headset in this application includes but is not limited to an in-ear headset, a semi-open headset, a head-mounted headset, a neck-mounted headset, and the like. This is not limited in this application. In addition, the mobile phone in FIG. 1a is merely an example in this application, and may be replaced with another terminal device such as a tablet computer, a notebook computer, or a smartwatch. This is not limited in this application.

FIG. 1b is a diagram of an example of a hearing loss simulation system. In FIG. 1b, the hearing loss simulation system may include a terminal device and a headset.

For example, the terminal device may include a hearing loss simulation application. The hearing loss simulation application may be configured to provide a user interaction interface. In this way, a user without hearing loss can experience, by inputting user information of a user with hearing loss and another interaction operation in the hearing loss simulation application, an effect of audio heard by the user with hearing loss.

For example, the headset may include a speaker, a hearing loss simulation apparatus, and a microphone. The microphone may be configured to collect audio data. The hearing loss simulation apparatus may be configured to perform, based on the user information of the user with hearing loss, hearing loss simulation on the audio data collected by the microphone or preset audio data (prestored in the terminal device, and sent by the terminal device to the headset), to simulate audio data heard by the user with hearing loss. The speaker may be configured to play the audio data heard by the user with hearing loss and simulated by the hearing loss simulation apparatus.

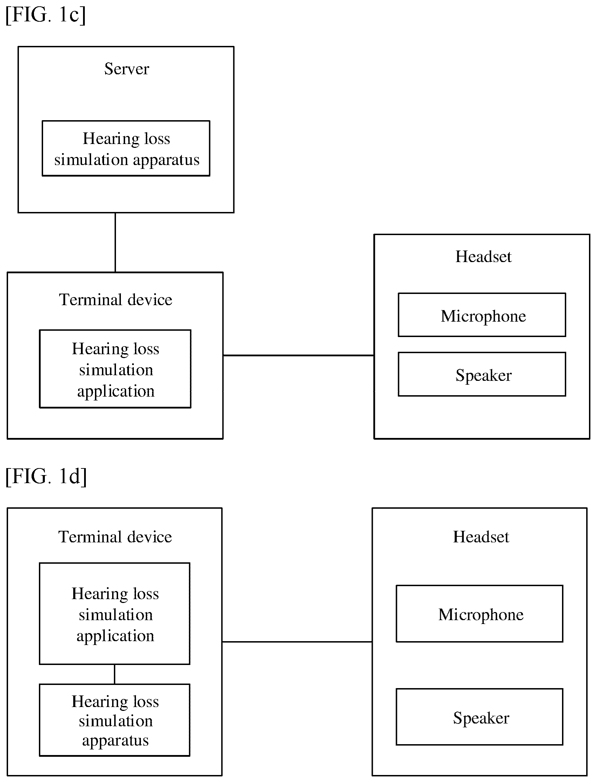

FIG. 1c is a diagram of an example of a hearing loss simulation system. In FIG. 1c, the hearing loss simulation system may include a server, a terminal device, and a headset.

For example, the terminal device may include a hearing loss simulation application. The hearing loss simulation application may be configured to provide a user interaction interface. In this way, a user without hearing loss can experience, by inputting user information of a user with hearing loss and another interaction operation in the hearing loss simulation application, an effect of audio heard by the user with hearing loss.

For example, the server may include a hearing loss simulation apparatus. The hearing loss simulation apparatus may be configured to perform, based on the user information of the user with hearing loss, hearing loss simulation on audio data (obtained by the terminal device from the headset and then sent to the server) collected by a microphone in the headset or preset audio data (prestored in the terminal device and sent by the terminal device to the server; or prestored in the server), to simulate audio data heard by the user with hearing loss.

For example, the server may be one server, or may be a server cluster including a plurality of servers, or may be another distributed system. This is not limited in this application.

For example, the headset may include a speaker and the microphone. The microphone may be configured to collect audio data, and the speaker may be configured to play audio data (obtained by the terminal device from the server and then sent to the headset) heard by the user with hearing loss and simulated by the hearing loss simulation apparatus in the server.

FIG. 1d is a diagram of an example of a hearing loss simulation system. In FIG. 1d, the hearing loss simulation system may include a terminal device and a headset.

For example, the terminal device may include a hearing loss simulation application and a hearing loss simulation apparatus. The hearing loss simulation application may be configured to provide a user interaction interface. In this way, a user without hearing loss can experience, by inputting user information of a user with hearing loss and another interaction operation in the hearing loss simulation application, an effect of audio heard by the user with hearing loss. The hearing loss simulation apparatus may be configured to perform, based on the user information of the user with hearing loss, hearing loss simulation on audio data (obtained by the terminal device from the headset) collected by a microphone in the headset or preset audio data (prestored in the terminal device), to simulate audio data heard by the user with hearing loss.

For example, the headset may include a speaker and the microphone. The microphone may be configured to collect audio data, and the speaker may be configured to play audio data heard by the user with hearing loss and simulated by the hearing loss simulation apparatus in the terminal device.

It should be understood that FIG. 1b, FIG. 1c, and FIG. 1d are merely examples in this application. The terminal device in this application may include more modules/applications than the terminal devices shown in FIG. 1b, FIG. 1c, and FIG. 1d. The headset in this application may include more modules/components than the headsets shown in FIG. 1b, FIG. 1c, and FIG. 1d. The server in this application may include more modules/components than the servers shown in FIG. 1b, FIG. 1c, and FIG. 1d. This is not limited in this application.

The following uses the hearing loss simulation system in FIG. 1b as an example to describe a process in which a user without hearing loss experiences an effect of audio heard by a user with hearing loss. For ease of subsequent description, the user without hearing loss is referred to as a first user, and the user with hearing loss is referred to as a second user. In addition, the headset is worn by the first user.

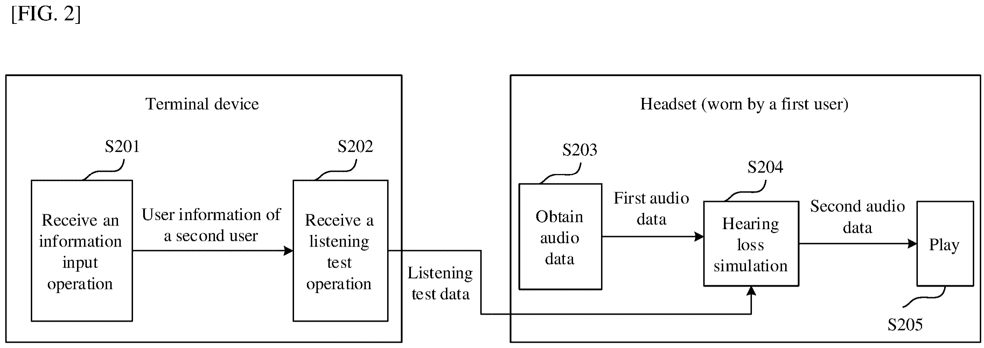

FIG. 2 is a diagram of an example of a hearing loss experience process.

S201: A terminal device receives an information input operation, and obtains user information of a second user that is correspondingly input by the information input operation.

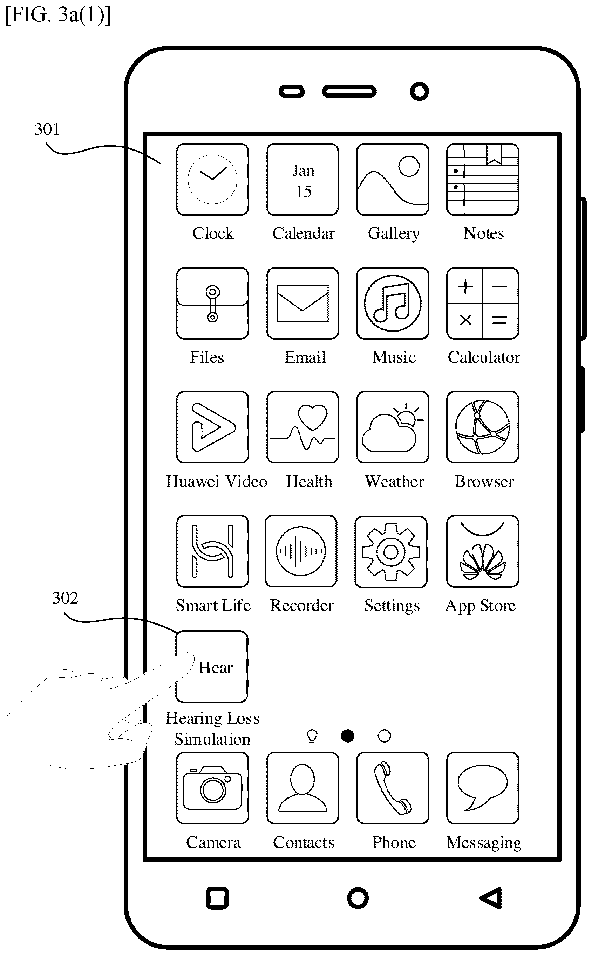

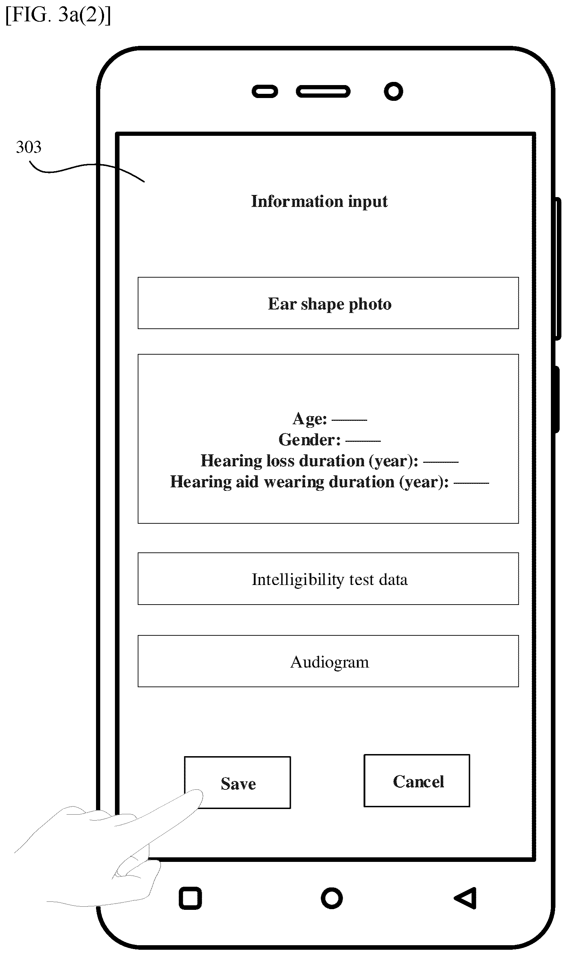

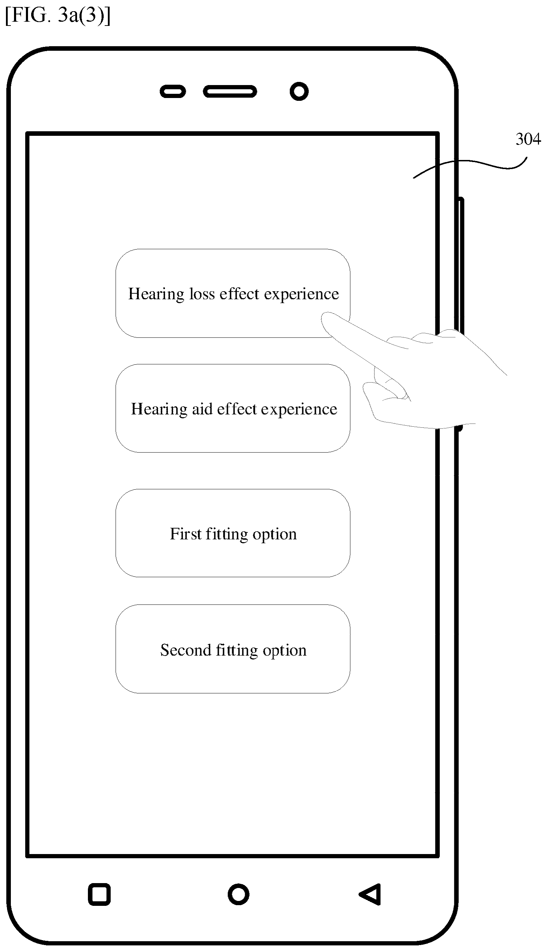

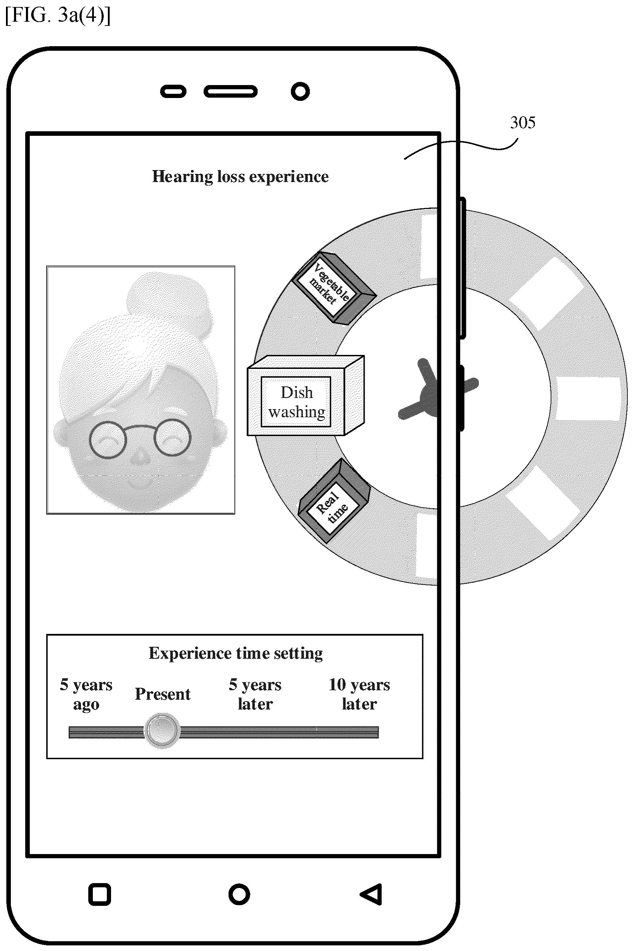

FIG. 3a(1) to FIG. 3a(4) are diagrams of examples of mobile phone interfaces.

With reference to FIG. 3a(1), for example, 301 is a home screen of the mobile phone, and the home screen of the mobile phone includes one or more controls, including but not limited to application icons (for example, an application icon of a browser application and an application icon 302 of a hearing loss simulation application), and the like.

With continued reference to FIG. 3a(1), for example, a user (the user is not limited to a user who interacts with the mobile phone, and may be a first user or the second user, and this application uses the first user as an example for description) taps the application icon 302 of the hearing loss simulation application, and the mobile phone may display an information input interface 303 in response to an operation of the first user, as shown in FIG. 3a(2). For example, the information input interface 303 includes one or more controls, including but not limited to: an ear shape photo option, an age option, a gender option, a hearing loss duration option, a hearing aid wearing duration option, an intelligibility test data option, an audiogram option, a save option, a cancel option, and the like. It should be understood that the information input interface 303 may further include other options or information. This is not limited in this application.

For example, the first user may tap the ear shape photo option to input (take or select from a gallery) an ear shape photo of the second user. For example, the first user may tap the age option to input an age of the second user. For example, the first user may tap the gender option to input a gender of the second user. For example, the first user may tap the hearing loss duration option to input hearing loss duration (which may be in units of years) of the second user. For example, the first user may tap the hearing aid wearing duration option to input hearing aid wearing duration (which may be in units of years) of the second user. For example, the first user may tap the intelligibility test data option to input intelligibility test data of the second user (a test may be performed to obtain intelligibility test data, or intelligibility test data obtained in advance by performing a test is uploaded). For example, the first user may tap the audiogram option to input an audiogram of the second user (a test may be performed to obtain an audiogram, or an audiogram obtained in advance by performing a test is uploaded).

For example, an operation of tapping each option on the information input interface 303 by the first user and an operation of inputting corresponding information may be referred to as an information input operation. For example, an operation of tapping the ear shape photo option and an operation of inputting the ear shape photo may be referred to as an information input operation. In this way, after receiving the information input operation of the first user, the terminal device may obtain the user information of the second user. The user information includes but is not limited to: the ear shape photo, the age, the gender, the hearing loss duration, the hearing aid wearing duration, the intelligibility test data, the audiogram, and the like.

It should be understood that the information input interface 303 may include any one or more of the ear shape photo option, the age option, the gender option, the hearing loss duration option, the hearing aid wearing duration option, the intelligibility test data option, and the audiogram option. Correspondingly, the user information may include any one or more pieces of the following information: the ear shape photo, the age, the gender, hearing loss duration, the hearing aid wearing duration, the intelligibility test data, and the audiogram.

S202: The terminal device receives a listening test operation, and sends listening test data to a headset in response to the listening test operation, where the listening test data includes the user information or hearing loss feature information, and the hearing loss feature information is determined based on the user information.

For example, after completing the information input, the first user may tap the save option on the information input interface 303, and the mobile phone may display a function selection interface 304 in response to an operation of the first user, as shown in FIG. 3a(3). For example, the function selection interface 304 includes one or more controls, including but not limited to a hearing loss effect experience option, a hearing aid effect experience option, a first fitting option, a second fitting option, and the like. It should be understood that the function selection interface 304 may further include other options or information. This is not limited in this application.

For example, the first user may tap the hearing loss effect experience option to experience a hearing loss effect, that is, experience an effect of audio heard by a user with hearing loss (that is, the second user). After the first user taps the hearing loss effect experience option (that is, performs a hearing loss effect experience operation), the mobile phone may receive the hearing loss effect experience operation. Then the mobile phone may display a hearing loss experience interface 305 in response to the hearing loss effect experience operation, as shown in FIG. 3a(4). For example, the hearing loss experience interface 305 includes one or more controls, including but not limited to: a scenario audio option (such as a dish washing scenario audio option, a vegetable market scenario audio option, or a real-time audio option), an experience time setting option, a cartoon image of the second user, and the like. It should be understood that the hearing loss experience interface 305 may further include other options or other information (for example, the user information of the second user, such as the gender, the age, and the hearing loss duration). This is not limited in this application. It should be noted that, in FIG. 3a(4), a part that is of a wheel and that is located outside the mobile phone is actually hidden. A complete wheel is shown in this application to indicate that a plurality of scenario audio options are included. In addition, the first user may select a corresponding scenario audio by rotating the wheel.

For example, the first user may select the dish washing scenario audio option on the hearing loss experience interface 305. In this way, the first user can experience an effect of audio heard by the second user in a dish washing scenario. For example, the first user may select the vegetable market scenario audio option. In this way, the first user can experience an effect of audio heard by the second user in a vegetable market. For example, the first user may select the real-time audio option. In this way, the first user can experience an effect of audio heard by the second user in real time in a current scenario.

For example, a selection operation performed by the first user on the scenario audio option may be referred to as a listening test operation. After the first user performs the listening test operation, the mobile phone may send the listening test data to the headset in response to the listening test operation.

For example, preset audio data corresponding to a preset audio scenario may be obtained in advance. The preset audio scenario may be a scenario in which the corresponding preset audio data can be obtained in advance, for example, the dish washing scenario or the vegetable market scenario, and does not include a real-time audio scenario. In this way, when the first user performs a selection operation for the scenario audio option (such as the dish washing scenario audio option or the vegetable market scenario audio option) corresponding to the preset audio scenario, the listening test data sent by the terminal device to the headset may include the preset audio data corresponding to the preset audio scenario selected by the first user.

For example, when the first user performs a selection operation for the scenario audio option corresponding to the real-time audio scenario, the listening test data sent by the terminal device to the headset may include an audio collection indication, where the audio collection indication indicates the headset to collect audio.

In addition, the listening test data sent by the terminal device to the headset may further include the user information of the second user or the hearing loss feature information, and the hearing loss feature information may be information used to determine a parameter required for a hearing loss simulation apparatus to perform hearing loss simulation, and may be determined based on the user information of the second user. For example, the hearing loss feature information may include but is not limited to ear-shaped features (such as an auricle length, an auricle width, an auricle depth, and an ear type), hearing loss frequency band information, an intelligibility score, an intelligibility confusion matrix, age-related decline information (such as the age, the gender, the hearing loss duration, and the hearing aid wearing duration), and the like. This is not limited in this application.

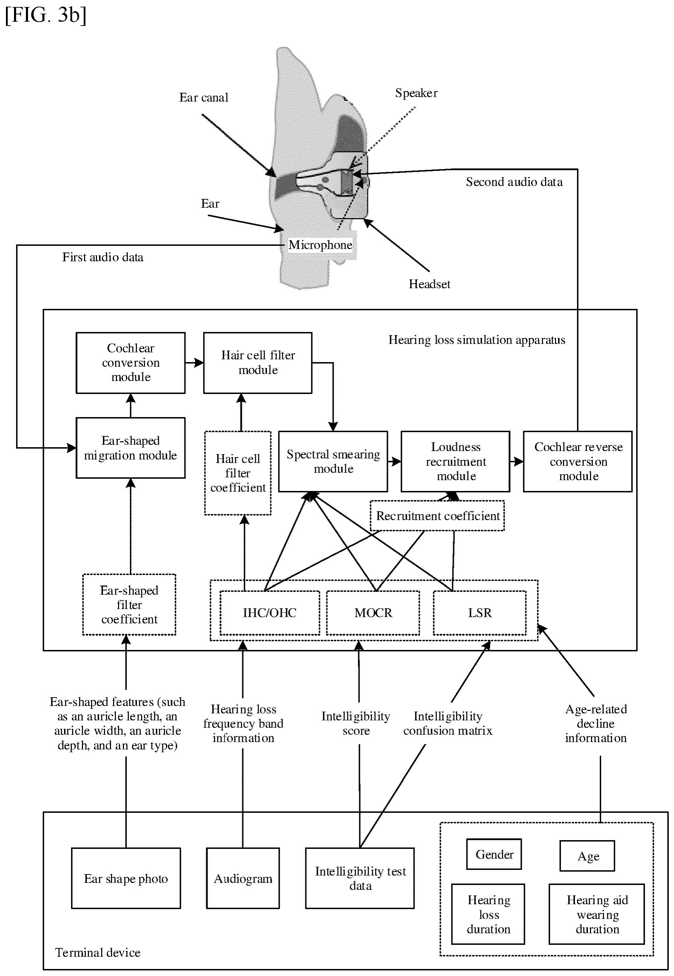

FIG. 3b is a diagram of an example of a hearing loss simulation system.

With reference to FIG. 3b, for example, ear-shaped features such as an auricle length, an auricle width, an auricle depth, and an ear type may be extracted by analyzing an ear shape photo. There may be a plurality of ear types, for example, cup-shaped ears, cauliflower ears, and protruding ears. This is not limited in this application. It should be understood that the ear-shaped features are not limited in this application.

With reference to FIG. 3b, for example, a degree of hearing loss (unit: dB) in each sub-band (that is, within different frequency ranges) may be extracted by analyzing an audiogram, and is used as hearing loss frequency band information.

With reference to FIG. 3b, for example, an intelligibility score and an intelligibility confusion matrix may be determined by analyzing intelligibility test data. A higher intelligibility score of audio data indicates that the audio data is easier to understand.

For example, a plurality of pieces of test audio data may be used to perform an intelligibility test on the second user, and then the intelligibility confusion matrix is determined based on the intelligibility test data.

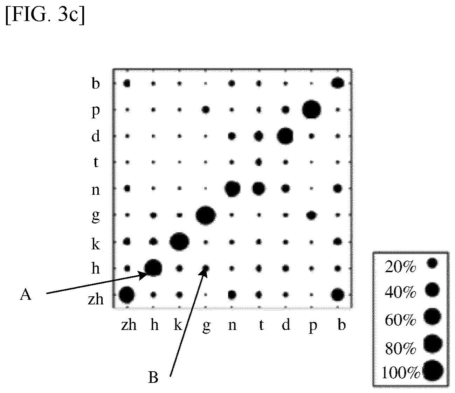

For example, if there are 50 pieces of test audio data, the obtained intelligibility confusion matrix may be shown in FIG. 3c. In FIG. 3c, a vertical coordinate is a syllable included in the test audio data, and a horizontal coordinate is a syllable heard by the second user. A circle A represents a statistical result of a syllable h in the test audio data that is heard by the second user as the syllable h (that is, a ratio of a quantity of times that the second user hears the syllable h to a quantity of words that are in the 50 pieces of test audio data and that include the syllable h; and a larger circle indicates a larger ratio). A circle B represents a statistical result of the syllable h in the test audio data that is heard by the second user as a syllable g. A larger circle on a diagonal of the intelligibility confusion matrix in FIG. 3c indicates a lower degree of hearing loss of the second user.

S203: The headset obtains first audio data.

For example, after receiving the listening test data, when it is determined that the listening test data includes the preset audio data, the headset may extract the preset audio data from the listening test data as the first audio data. When it is determined that the listening test data includes the audio collection indication, a microphone in the headset may be started in response to the audio collection indication. In this way, the microphone collects real-time audio data, and the real-time audio data collected by the microphone is used as the first audio data.

S204: The headset performs hearing loss simulation on the first audio data based on the hearing loss feature information, to obtain second audio data.

For example, when the listening test data includes the hearing loss feature information, the headset may perform hearing loss simulation on the first audio data based on the hearing loss feature information, to obtain the second audio data. When the listening test data includes the user information, the headset may determine the hearing loss feature information based on the user information, and then perform hearing loss simulation on the first audio data based on the hearing loss feature information, to obtain the second audio data. In other words, this application does not limit whether the terminal device determines the hearing loss feature information based on the user information, or the headset determines the hearing loss feature information based on the user information.

For example, the headset may invoke the hearing loss simulation apparatus to perform hearing loss simulation on the first audio data based on the hearing loss feature information, to obtain the second audio data. For example, the hearing loss simulation apparatus may determine a hearing loss simulation parameter based on the hearing loss feature information; and then perform hearing loss simulation on the first audio data based on the hearing loss simulation parameter, to obtain the second audio data.

For example, a cochlea (which may include hair cells (including inner hair cells (inner hear cells, IHCs) and outer hair cells (outer hear cells, OHCs))) that can modulate audio is one of key elements that enable a user to clearly hear audio content. Therefore, a hair cell filter module (or referred to as a hair cell filter group (hair cell filter group)) configured to simulate audio modulation by the IHCs and the OHCs may be disposed in the hearing loss simulation apparatus, and the hair cell filter module is used to perform hair cell filtering processing on the first audio data, to obtain the second audio data.

For example, the hearing loss simulation parameter may include a hair cell filter coefficient. Specific quantities of the IHCs and the OHCs (or a ratio of a quantity of the IHCs to a quantity of the OHCs) may be determined based on the hearing loss frequency band information, the intelligibility score, the intelligibility confusion matrix, and the age-related decline information; and then the hair cell filter coefficient is determined based on the specific quantities of the IHCs and the OHCs (or the ratio of the quantity of the IHCs to the quantity of the OHCs). Then the hair cell filter module may perform hair cell filtering processing on the first audio data based on the hair cell filter coefficient, to obtain the second audio data.

With reference to FIG. 3b again, for example, the hearing loss simulation apparatus includes an ear-shaped migration (Ear-shaped feature simulation) module, a cochlear conversion (Cochlear conversion) module, a hair cell filter module, a spectral smearing (spectral smearing) module, a loudness recruitment (loudness recruitment) module, and a cochlear reverse conversion (cochlear reverse Conversion) module.

It should be noted that, in the embodiment in FIG. 2, the hearing loss simulation apparatus is located in the headset, and the hearing loss simulation apparatus may be implemented based on a DSP (Digital signal processor, digital signal processor) in the headset.

For example, the ear-shaped migration module may be configured to perform ear-shaped migration processing based on an ear-shaped feature. Specifically, the hearing loss simulation parameter may further include an ear-shaped filter coefficient. The headset or the terminal device may determine the ear-shaped filter coefficient based on the ear-shaped feature.

In a possible manner, the headset or the terminal device may determine an ear-shaped filter parameter based on the ear-shaped feature determined by the ear shape photo of the second user. In a possible manner, an ear shape photo of the first user may also be input on the information input interface 303, and a corresponding ear-shaped feature is determined. In this case, the headset or the terminal device may determine the ear-shaped filter parameter based on the ear-shaped feature determined by the ear shape photo of the second user and the ear-shaped feature determined by the ear shape photo of the first user. For example, a correlation between the ear-shaped feature determined based on the ear shape photo of the second user and the ear-shaped feature determined based on the ear shape photo of the first user may be determined. Then the ear-shaped filter parameter is determined based on the correlation.

Then the ear-shaped migration module in the headset may perform filtering processing (or referred to as modulation) on the first audio data based on the ear-shaped filter coefficient, to obtain first intermediate audio data. In a possible manner, a plurality of groups of preset ear-shaped filter coefficients may be preset, and then an ear-shaped filter coefficient that matches the ear-shaped feature is selected from the plurality of groups of preset ear-shaped filter coefficients. In a possible manner, calculation may be performed based on the ear-shaped feature, to determine the ear-shaped filter coefficient.

For example, the cochlear conversion module may be configured to perform signal processing in a manner similar to a cochlear physiological mechanism. Specifically, the cochlear conversion module may perform filtering processing on energy distribution of the first intermediate audio data in each frequency band by using a cochlear conversion filter coefficient, so that an energy distribution feature of filtered first intermediate audio data (which is subsequently referred to as second intermediate audio data) approximates to an energy distribution feature obtained by processing a standard cochlear signal.

For example, the hair cell filter module may be configured to perform hair cell filtering on the second intermediate audio data, to obtain third intermediate audio data.

For example, the spectral smearing module may be configured to perform spectral smearing, that is, first perform frequency domain transformation on the third intermediate audio data, and then reduce and randomize time-frequency resolution in a time-frequency region of the third intermediate audio data, to obtain fourth intermediate audio data. For example, the headset may determine a medial olivocochlear reflex (medial olivocochlear reflex, MOCR) parameter based on the hearing loss frequency band information, the intelligibility score, the intelligibility confusion matrix, and the age-related decline information, and determine a parameter indicating distribution of auditory nerve fibers of low, medium, and high spontaneous rates (low, medium, and high spontaneous rate, LSR). Then the spectral smearing module may perform spectral smearing based on at least one of the specific quantities of the IHCs and the OHCs (or the ratio of the quantity of the IHCs to the quantity of the OHCs is determined), the MOCR parameter, and the LSR parameter.

For example, the loudness recruitment module may perform filtering processing based on a recruitment coefficient. Specifically, the loss simulation parameter may further include the recruitment coefficient. The headset may determine the MOCR parameter and the LSR parameter based on the hearing loss frequency band information, the intelligibility score, the intelligibility confusion matrix, and the age-related decline information. Then the recruitment coefficient is determined based on at least one of the specific quantities of the IHCs and the OHCs (or the ratio of the quantity of the IHCs to the quantity of the OHCs is determined), the MOCR parameter, and the LSR parameter. Then the loudness recruitment module performs filtering processing on the fourth intermediate audio data based on the recruitment coefficient, to obtain fifth intermediate audio data.

For example, the cochlear reverse conversion module may be configured to perform signal processing on an audio signal in a manner similar to a reverse cochlear physiological mechanism, to achieve an objective of outputting a sound signal that is heard by a patient with hearing loss. The process may be reverse to that of the cochlear conversion module. Specifically, the cochlear reverse conversion module may perform filtering processing on the fifth intermediate audio data by using a cochlear reverse conversion filter coefficient, so that an energy distribution feature of filtered fifth intermediate audio data (that is, the second audio data) approximates to an energy distribution feature obtained by performing reverse processing on a standard cochlear signal.

As can be learned from the foregoing description, an actual auditory pathway such as inner/outer hair cells and cochlear reflexes of an ear is considered, and impact of audio intelligibility, a degree of hearing loss, an age, and the like on physiological parameters in the auditory pathway is considered. Therefore, compared with the conventional technology, the hearing loss simulation of the hearing loss simulation apparatus in this application better conforms to a physiological process of an auditory periphery, and can simulate audio data closer to audio data heard by a user with hearing loss.

It should be understood that the hearing loss simulation apparatus may further include more or fewer modules than those shown in FIG. 3b. This is not limited in this application.

S205: The headset plays the second audio data.

Then the headset may send the second audio data to a speaker, and the speaker plays the second audio data. In this way, the first user can hear, by wearing the headset, the audio data heard by the second user, and experience an effect of the audio data heard by the second user.

In this way, with reference to the foregoing manner, the first user can select different scenario audio options on the hearing loss experience interface 305 in FIG. 3a(4), to experience audio data heard by the second user in different scenarios.

In a family scenario, both the first user and the second user may be family members, the second user is a grandfather, and the first user is a grandson. After experiencing an effect of audio heard by the grandfather, and perceiving feelings of the grandfather and inconveniences caused by hearing loss in life, the grandson can be prompted to buy a hearing aid for the grandfather; and the grandson can be more patient when communicating with the grandfather, and in a process of communicating with the grandfather, speak louder, repeat words more frequently, and reduce a speaking speed.

In another scenario, for example, in a working scenario, the first user is an employee of a human resources department, and the second user is an employee of a logistics department. After experiencing an effect of audio data heard by the employee of the logistics department, the employee of the human resources department can perceive feelings of the employee of the logistics department and inconveniences caused by hearing loss in life and work. In this way, the employee of the human resources department can be prompted to be more patient when communicating with the employee of the logistics department, and in a process of communicating with the employee of the logistics department, speak louder, repeat words more frequently, and reduce a speaking speed.

With reference to FIG. 3a(4) again, for example, the experience time setting option on the hearing loss experience interface 305 may be a sliding control. The sliding control may include a slider bar, a slider knob, and scale marks, and each scale mark is a time point. The sliding control in FIG. 3a(4) includes four scale marks: 5 years ago, present, 5 years later, and 10 years later. When the first user slides the slider knob in the sliding control to a scale mark, the first user may experience an effect of audio heard by the second user at an age corresponding to the scale mark. For example, after the first user slides the slider knob in the sliding control corresponding to the experience time setting option to a scale mark (that is, performs an experience time setting operation), the mobile phone may receive the experience time setting operation, and determine a time point corresponding to the scale mark at which the slider knob is located; and then determine that the time point corresponding to the scale mark at which the slider knob is located is experience time information (that is, obtain the experience time information). For example, if the time point corresponding to the scale mark is 5 years ago, experience time information is -5 years; if the time point corresponding to the scale mark is present, experience time information is 0 years; if the time point corresponding to the scale mark is 5 years later, experience time information is +5 years; or if the time point corresponding to the scale mark is 10 years later, experience time information is +10 years. In this case, the age in the age-related decline information may be a sum of the age in the user information and the experience time information. In other words, in this case, the hearing loss feature information may be determined based on the user information and the experience time information. In this case, the listening test data may further include the experience time information.

For example, after receiving the listening test operation, the terminal device may further display, on the hearing loss experience interface 305, text information corresponding to the first audio data, and annotate a target word in the text information based on the second audio data, where an intelligibility score of the target word is less than an intelligibility threshold, and/or the target word is a word whose sound quality score is less than a sound quality threshold. In this way, the first user can conveniently learn audio of words that the second user cannot hear or cannot clearly hear. The intelligibility threshold and the sound quality threshold may be set based on a requirement. This is not limited in this application.

For example, because the terminal device prestores the preset audio data, the terminal device may perform speech recognition in advance on the preset audio data, to determine corresponding text information. Further, after receiving a selection operation for a preset scenario audio option, the terminal device may display the text information corresponding to the first audio data. Certainly, after receiving the selection operation for the preset scenario audio option, the terminal device may also perform speech recognition on the first audio data, to determine and display the corresponding text information.

For example, after receiving a selection operation for the real-time audio option, the terminal device may obtain the first audio data from the headset, and then perform speech recognition on the first audio data, to determine and display the corresponding text information.

For example, the terminal device may obtain the second audio data from the headset, and then perform intelligibility scoring and sound quality scoring on the second audio data, to determine an intelligibility score and a sound quality score of each word. Then the terminal device may determine that a word whose intelligibility score is less than the intelligibility threshold and/or whose sound quality score is less than the sound quality threshold is the target word, and annotate the target word in the text information corresponding to the first audio data.

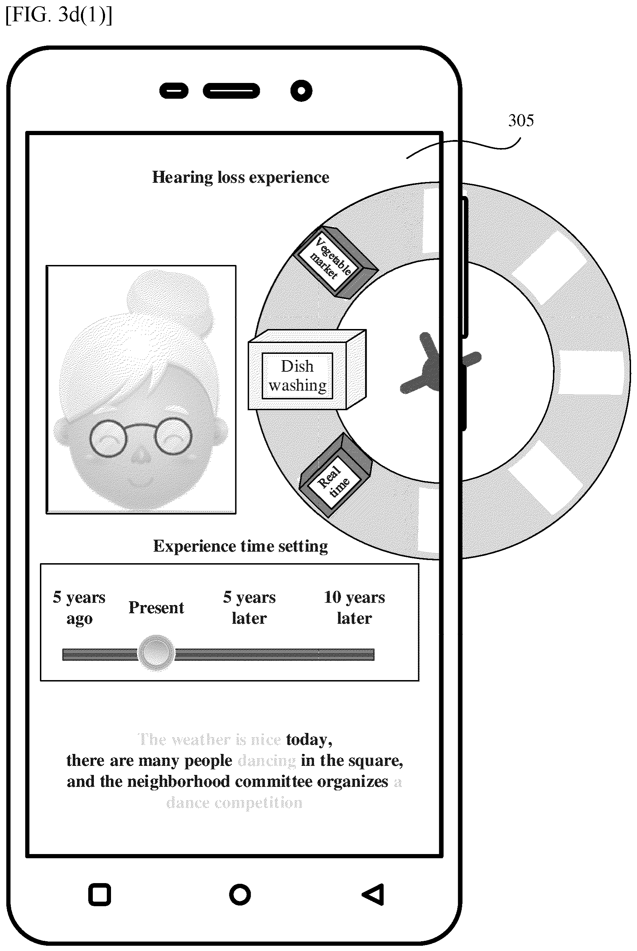

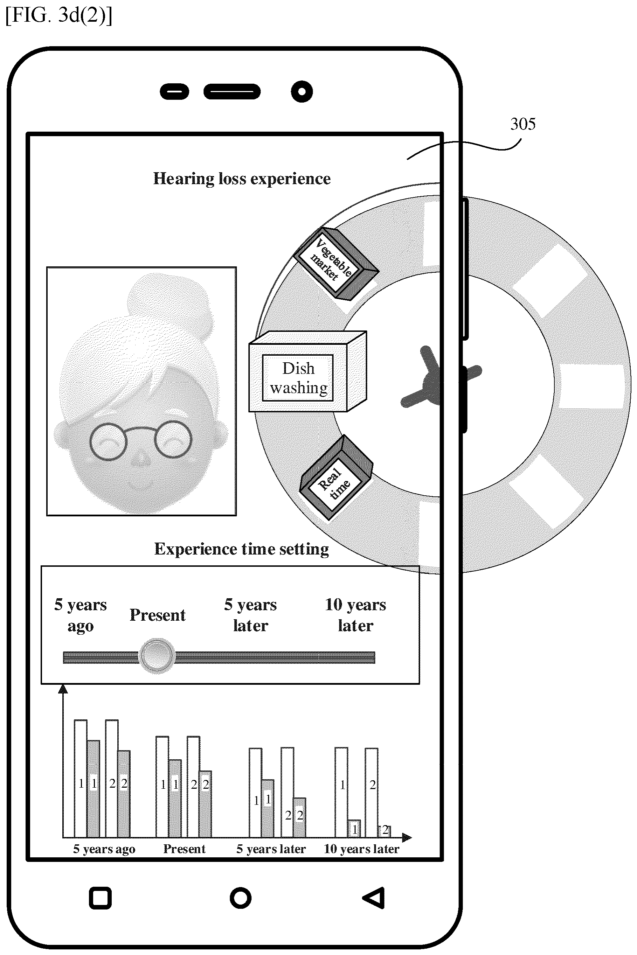

FIG. 3d(1) and FIG. 3d(2) are diagrams of examples of mobile phone interfaces. The text information corresponding to the first audio data on the hearing loss experience interface 305 in FIG. 3d(1) is "The weather is nice today, there are many people dancing in the square, and the neighborhood committee organizes a dance competition", where target words include "The weather is nice", "dance", and "dance competition". In this case, the target words may be annotated by using another color. As shown in FIG. 3d(1), the text information corresponding to the first audio data is black, and the target words are gray. It should be understood that the target word may be annotated in a plurality of manners, for example, in a manner of adding an underline or using boldface. This is not limited in this application.

For example, the terminal device may perform objective evaluation on the first audio data to obtain first evaluation information; and perform objective evaluation on the second audio data to determine second evaluation information; and display the first evaluation information and the second evaluation information, where the first evaluation information includes a first intelligibility score and/or a first sound quality score, and the second evaluation information includes a second intelligibility score and/or a second sound quality score. In this way, a hearing gap between a normal person and a person with hearing loss can be quantified, which better helps the second user feel inconveniences in life of the first user. Therefore, care is enhanced, and there is greater motivation to buy a hearing aid subsequently.

With reference to FIG. 3d(2), for example, the first intelligibility score and the first sound quality score are displayed on the hearing loss experience interface 305 in FIG. 3d(2), as shown in white rectangles, where "1" in a white rectangle represents the first intelligibility score, and "2" in a white rectangle represents the first sound quality score. The second intelligibility score and the second sound quality score are displayed, as shown in gray rectangles, where "1" in a gray rectangle represents the second intelligibility score, and "2" in a gray rectangle represents the second sound quality score.

For example, the terminal device may first perform objective evaluation on the first audio data to obtain initial evaluation information, and then correct the initial evaluation information based on the experience time information to obtain the first evaluation information. For example, a mapping relationship between experience time information and a correction factor is pre-established. For example, the mapping relationship is {(0, 1), (+5, 0.85), (+10, 0.75), (-5, 1.15)}. (0, 1) indicates that when the experience time information is 0, a corresponding correction factor is 1; (+5, 0.85) indicates that when the experience time information is +5, a corresponding correction factor is 0.58; (+10, 0.75) indicates that when the experience time information is +10, a corresponding correction factor is 0.75; and (-5, 1.15) indicates that when the experience time information is -5, a corresponding correction factor is 1.15. Then a product value of the initial evaluation information and the correction factor may be used as the first evaluation information.

For example, when the first user slides the slider knob in the sliding control to the scale mark "present", the first user may display, on the hearing loss experience interface 305, a second intelligibility score and a second sound quality score that correspond to the present, and display a first intelligibility score and a first sound quality score that correspond to the present.

For example, when the first user slides the slider knob in the sliding control to the scale mark "5 years ago", the first user may display, on the hearing loss experience interface 305, a second intelligibility score and a second sound quality score that correspond to 5 years ago, and display a first intelligibility score and a first sound quality score that correspond to 5 years ago.

For example, when the first user slides the slider knob in the sliding control to the scale mark "5 years later", the first user may display, on the hearing loss experience interface 305, a second intelligibility score and a second sound quality score that correspond to 5 years later, and display a first intelligibility score and a first sound quality score that correspond to 5 years later.

For example, when the first user slides the slider knob in the sliding control to the scale mark "10 years later", the first user may display, on the hearing loss experience interface, a second intelligibility score and a second sound quality score that correspond to 10 years later, and display a first intelligibility score and a first sound quality score that correspond to 10 years later.

For example, the headset may further play the first audio data before or after playing the second audio data. In this way, the first user can conveniently compare audio data heard by a normal person with audio data heard by a user with hearing loss, to better experience a difference between the audio data heard by the normal person and the audio data heard by the user with hearing loss.

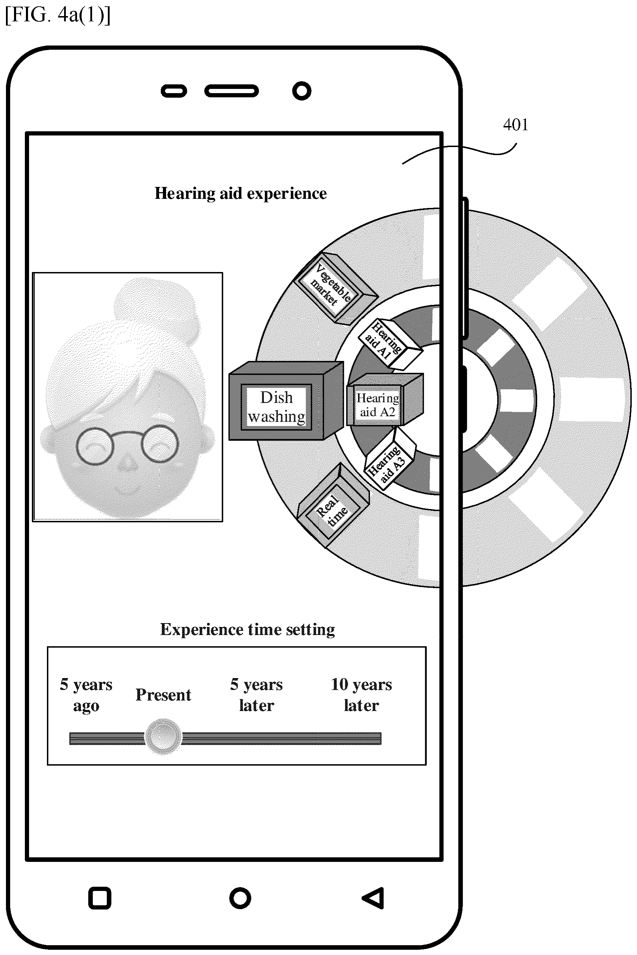

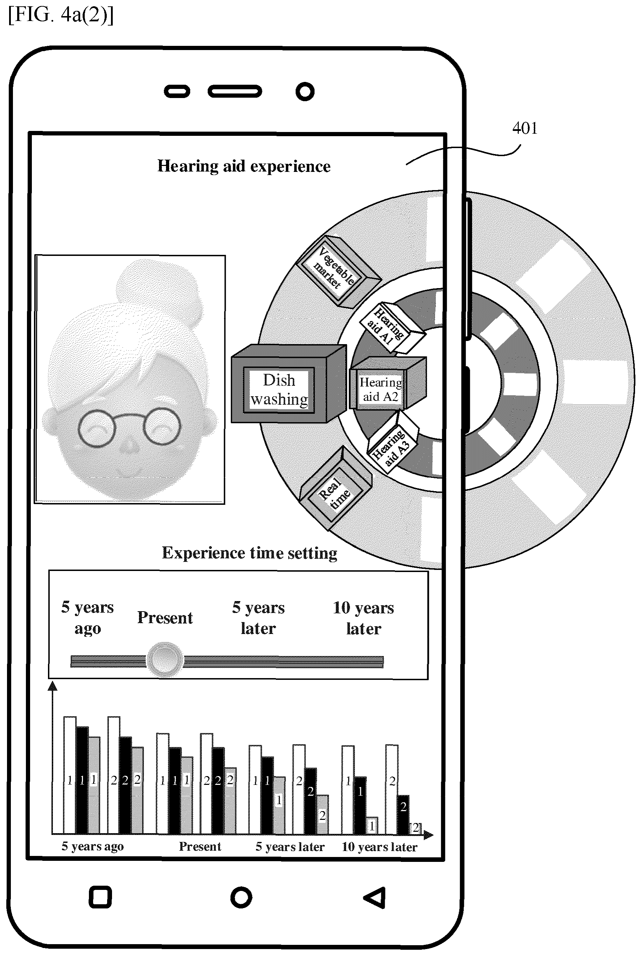

FIG. 4a(1) and FIG. a(2) are diagrams of examples of mobile phone interfaces.

With reference to FIG. 3a(2) again, for example, the first user may tap the hearing aid effect experience option to experience a hearing aid effect, that is, experience an effect of audio heard by the second user after the second user uses a hearing aid, to configure an appropriate hearing aid type for the second user. After the first user taps the hearing aid effect experience option (that is, performs a hearing aid effect experience operation), the mobile phone may receive the hearing aid effect experience operation. Then the mobile phone may display a hearing aid experience interface 401 in response to the hearing aid effect experience operation, as shown in FIG. 4a(1). With reference to FIG. 4a(1), the hearing aid experience interface 401 may include one or more controls, including but not limited to: a scenario audio option (such as a dish washing scenario audio option, a vegetable market scenario audio option, or a real-time audio option), an experience time setting option, a cartoon image of the second user, and a hearing aid type option (such as a hearing aid A1, a hearing aid A2, or a hearing aid A3, where A1, A2, and A3 are models of hearing aids). It should be understood that the hearing aid experience interface 401 may further include other options or other information (for example, the user information of the second user, such as the gender, the age, and the hearing loss duration). This is not limited in this application.

For example, on the hearing aid experience interface 401, the first user may first perform a selection operation (that is, perform a hearing aid type selection operation) for one hearing aid type option from a plurality of hearing aid type options. In this case, the mobile phone may obtain hearing aid type information corresponding to the first selected hearing aid type option. Then the first user may slide a slider knob in a sliding control corresponding to the experience time setting option to a scale mark (that is, perform an experience time setting operation). In this case, the mobile phone may obtain experience time information (optional step). Then the first user may perform a selection operation (that is, a listening test operation) for one scenario audio option from a plurality of scenario audio options. In this case, the mobile phone may send listening test data to the headset in response to the listening test operation, that is, perform S202. In this case, the listening test data may further include the hearing aid type information. It should be understood that a sequence of performing the hearing aid type selection operation and the experience time setting operation by the first user is not limited in this application.

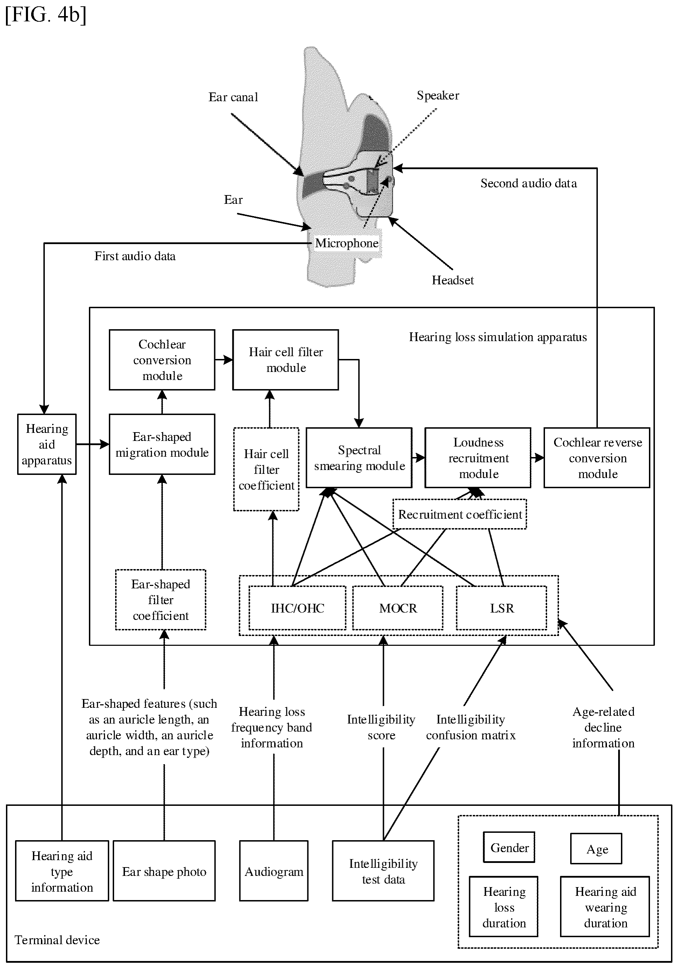

FIG. 4b is a diagram of an example of a hearing loss simulation system. FIG. 4b is shown on a basis of FIG. 3b. A hearing aid apparatus may be located in the headset, and the hearing aid apparatus may be implemented based on the DSP of the headset. The hearing aid apparatus may be configured to invoke a hearing aid algorithm to perform hearing aid optimization processing on audio data, so as to improve intelligibility and sound quality of audio.

With reference to FIG. 4b, for example, after receiving the listening test data, the headset may obtain the first audio data, and when determining that the listening test data includes the hearing aid type information, the headset may start the hearing aid apparatus. In this case, the hearing aid apparatus may invoke a hearing aid algorithm corresponding to the hearing aid type information to perform hearing aid optimization processing on the first audio data, to obtain third audio data, and output the third audio data to the hearing loss simulation apparatus. Then the hearing loss simulation apparatus performs hearing loss simulation processing on the third audio data based on the hearing loss feature information, to obtain the second audio data. Refer to the foregoing description. Details are not described herein again.

For example, the headset may be further configured to: perform hearing loss simulation on the first audio data based on the hearing loss feature information, to obtain sixth audio data; and then play the sixth audio data. In this way, the first user may compare an effect of audio that is heard by the second user by using no hearing aid with an effect of audio that is heard by the second user by using a hearing aid, to understand an effect of the hearing aid on the second user, so that the first user is further prompted to buy a hearing aid for the second user.

In a possible manner, a hearing aid switch may be set on the hearing aid experience interface 401. By default, the hearing aid switch is in an on state. In this case, the headset plays only the first audio data and the second audio data. When the first user needs to compare the second audio data obtained by performing hearing aid optimization and hearing loss simulation with the sixth audio data obtained by performing only hearing loss simulation, the first user may set the hearing aid switch to an off state. In this case, the headset plays only the first audio data and the sixth audio data.

In a possible manner, alternatively, the hearing aid switch may not be set on the hearing aid experience interface 401. In this way, the headset may sequentially play the first audio data, the second audio data, and the sixth audio data.

For example, the headset may send the sixth audio data to the terminal device. After receiving the sixth audio data, the terminal device may perform objective evaluation on the sixth audio data, to obtain third evaluation information. The third evaluation information may include a third intelligibility score and/or a third sound quality score. For example, the terminal device may further display the first evaluation information, the second evaluation information, and the third evaluation information on the hearing aid experience interface 401. For details, refer to the foregoing description. Details are not described herein again.

With reference to FIG. 4a(2), for example, the first intelligibility score and the first sound quality score are displayed on the hearing aid experience interface 401 in FIG. 4a(2), as shown in white rectangles, where "1" in a white rectangle represents the first intelligibility score, and "2" in a white rectangle represents the first sound quality score. The second intelligibility score and the second sound quality score are displayed, as shown in black rectangles, where "1" in a black rectangle represents the second intelligibility score, and "2" in a black rectangle represents the second sound quality score. The third intelligibility score and the third sound quality score are displayed, as shown in gray rectangles, where "1" in a black rectangle represents the third intelligibility score, and "2" in a black rectangle represents the third sound quality score.

In this way, the first user may select different hearing aid type options on the hearing aid experience interface 401 in FIG. 4a(1), to experience effects of audio heard by the second user when the second user wears different types of hearing aids; and with reference to subjective perception and objective evaluation information (which may include the first evaluation information, the second evaluation information, and the third evaluation information), select a hearing aid type with an optimal effect, to configure a most appropriate hearing aid type for the first user.

In a possible manner, the headset may be used as a hearing aid. After determining the hearing aid type with the optimal effect, the first user may select a corresponding hearing aid type option on the hearing aid experience interface 401 in FIG. 4a(1). In this way, in a subsequent process in which the second user uses the headset as a hearing aid, the hearing aid apparatus in the headset may invoke a hearing aid algorithm corresponding to the hearing aid type option to perform hearing aid optimization processing, and send the third audio data obtained through the hearing aid optimization processing to the speaker for playing (in this case, the hearing loss simulation apparatus does not run).

In a possible manner, after determining the hearing aid type with the optimal effect, the first user may buy a hearing aid based on the hearing aid type with the optimal effect.

It should be noted that, in the embodiments in FIG. 4a and FIG. 4b, in addition to the second intelligibility score and the second sound quality score, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

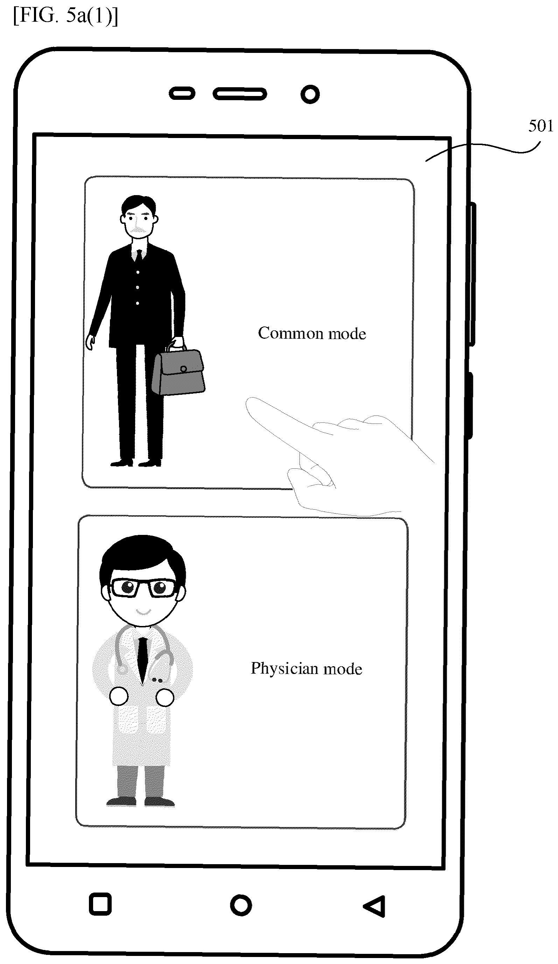

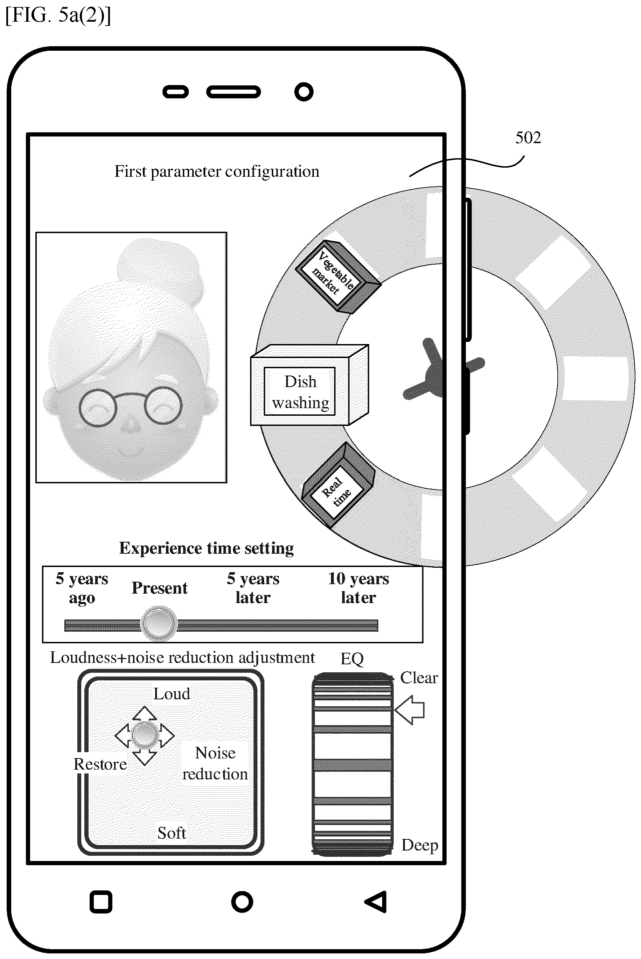

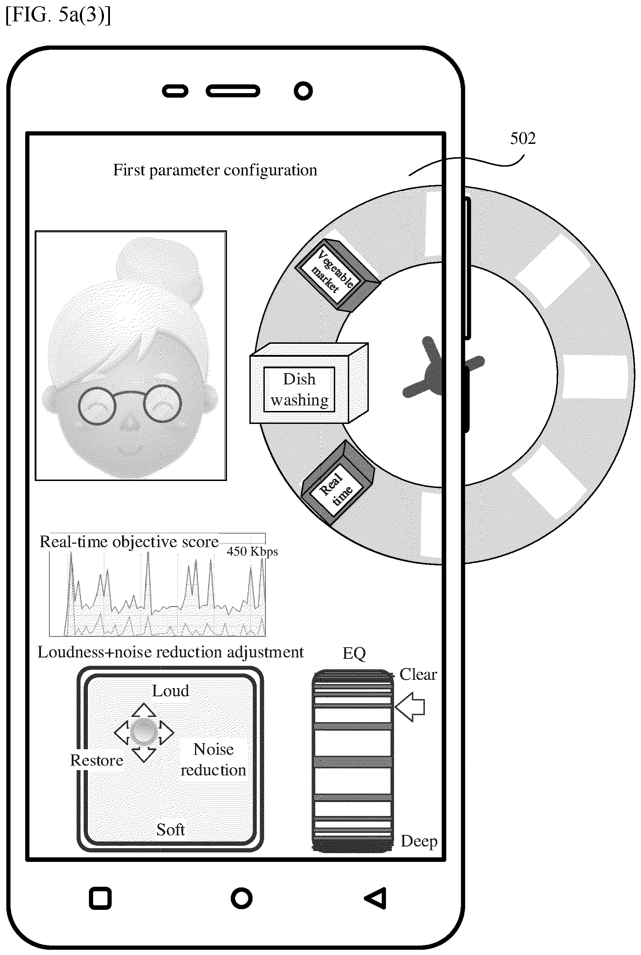

FIG. 5a(1) to FIG. 5a(3) are diagrams of examples of mobile phone interfaces.

With reference to FIG. 3a(3) again, for example, the first user may tap the first fitting option to fit a hearing aid algorithm parameter for the second user. For example, a professional physician and a common user have different knowledge in the audio field. Therefore, hearing aid algorithm parameters to be fitted for the professional physician and the common user may be different. After the first user taps the first fitting option (that is, performs a first fitting operation), the mobile phone may receive the first fitting operation. Then the mobile phone may display an identity selection interface 501 in response to the first fitting operation, as shown in FIG. 5a(1). With reference to FIG. 5a(1), the identity selection interface 501 may include one or more controls, including but not limited to a physician mode option and a common mode option. This is not limited in this application.

For example, when the first user is a common user (a non-professional physician), the common mode may be selected on the identity selection interface 501. After the user taps the common mode option (that is, performs a first identity selection operation), the mobile phone may display a first parameter configuration interface 502 in response to the operation of the first user, as shown in FIG. 5a(2). With reference to FIG. 5a(2), the first parameter configuration interface 502 may include one or more controls, including but not limited to: a scenario audio option (such as a dish washing scenario audio option, a vegetable market scenario audio option, and a real-time audio option), an experience time setting option, a cartoon image of the second user, a first parameter configuration item (such as a noise reduction strength configuration item, a gain (loudness) configuration item, and an equalization (Equalisation, EQ) configuration item), and the like. It should be understood that the first parameter configuration interface 502 may further include other options or other information (for example, the user information of the second user, such as the gender, the age, and the hearing loss duration). This is not limited in this application.

It should be noted that, because noise reduction and loudness interact with and affect each other and jointly influence an effect of a hearing aid, in FIG. 5a(2), the noise reduction strength configuration item and the loudness configuration item are coupled into one control (for example, a loudness+noise reduction adjustment control in FIG. 5a(2)). In another embodiment, the noise reduction strength configuration item and the loudness configuration item may be two controls independent of each other. This is not limited in this application.

With reference to FIG. 5a(2), for example, the user may adjust a position of a virtual operation button in the loudness+noise reduction adjustment control to adjust loudness and noise reduction strength. Correspondingly, the mobile phone may obtain the corresponding noise reduction strength and loudness by reading horizontal and vertical coordinates of the virtual operation button in the loudness+noise reduction adjustment control.

With reference to FIG. 5a(2), for example, on the first parameter configuration interface 502, the first user may first slide a slider knob in a sliding control corresponding to the experience time setting option to a scale mark. In this case, the mobile phone may obtain experience time information. Then one or more first parameter configuration items may be set (that is, a first parameter setting operation is performed). In this case, the mobile phone may obtain a first hearing aid algorithm parameter corresponding to a first identity. For a first parameter configuration item currently set by the first user, a first hearing aid algorithm parameter currently set by the first user may be obtained. For a first parameter configuration item currently not set by the first user, a first hearing aid algorithm parameter historically set by the first user or a default first hearing aid algorithm parameter may be obtained. The first hearing aid algorithm parameter corresponding to the first identity is a part of a second hearing aid algorithm parameter (the second hearing aid algorithm parameter is all parameters required for calculation of the hearing aid algorithm). Then the first user may select a scenario audio option (that is, perform a listening test operation) from a plurality of scenario audio options. In this case, the mobile phone may send listening test data to the headset in response to the listening test operation, that is, perform S202. In this case, the listening test data may further include the first hearing aid algorithm parameter corresponding to the first identity. The first hearing aid algorithm parameter corresponding to the first identity may include but is not limited to noise reduction strength, gain, EQ, and the like. This is not limited in this application.

Then, after receiving the listening test data, the headset may obtain the first audio data, and when determining that the listening test data includes the first hearing aid algorithm parameter, the headset may start the hearing aid apparatus, and the hearing aid apparatus performs hearing aid optimization processing. Specifically, the hearing aid apparatus performs hearing aid optimization processing on the first audio data based on the first hearing aid algorithm parameter and other hearing aid algorithm parameters (parameters other than the first hearing aid algorithm parameter among all parameters required by the hearing aid algorithm) by using the hearing aid algorithm, to obtain fourth audio data and output the fourth audio data to the hearing loss simulation apparatus. Then the hearing loss simulation apparatus performs hearing loss simulation processing on the fourth audio data based on the hearing loss feature information, to obtain the second audio data. Refer to the foregoing description. Details are not described herein again.

For example, the terminal device may further display objective evaluation information in real time on the first parameter configuration interface 502, for example, a curve shown in a real-time objective score box in FIG. 5a(3). The objective evaluation information is determined based on an intelligibility score and a sound quality score, and in the real-time objective score box in 5a(3), a curve located above is determined based on the fourth audio data obtained through hearing aid optimization processing and output by the hearing aid apparatus, and a curve located below is determined based on the second audio data output by the hearing loss simulation apparatus (that is, determined based on the second evaluation information). In addition to the second intelligibility score and the second sound quality score, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

In this way, with reference to the foregoing manner, the first user may perform different configurations for the first parameter configuration item on the first parameter configuration interface 502 in FIG. 5a(2), to experience audio data heard by the second user after the second user wears hearing aids with different hearing aid algorithm parameters; and select an optimal first hearing aid algorithm parameter with reference to subjective perception and objective evaluation information (which may include the second evaluation information), to configure an appropriate hearing aid algorithm parameter for the first user.

In a possible manner, the headset may be used as a hearing aid. After determining the first hearing aid algorithm parameter with an optimal effect, the first user may configure the first parameter configuration item on the first parameter configuration interface 502 in FIG. 5a(2) based on the optimal first hearing aid algorithm parameter. In this way, in a subsequent process in which the second user uses the headset as a hearing aid, the hearing aid algorithm of the hearing aid apparatus in the headset may perform hearing aid optimization processing by using the first hearing aid algorithm parameter with the optimal effect and other hearing aid algorithm parameters, and send the fourth audio data obtained through the hearing aid optimization processing to the speaker for playing (in this case, the hearing loss simulation apparatus does not run).

In a possible manner, after determining the first hearing aid algorithm parameter with the optimal effect, the first user may buy a hearing aid based on the first hearing aid algorithm parameter with the optimal effect.

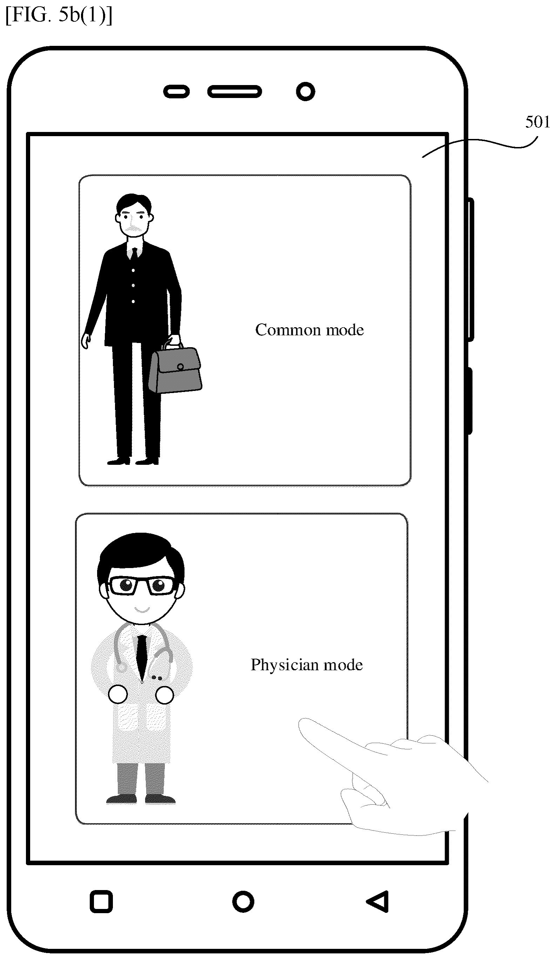

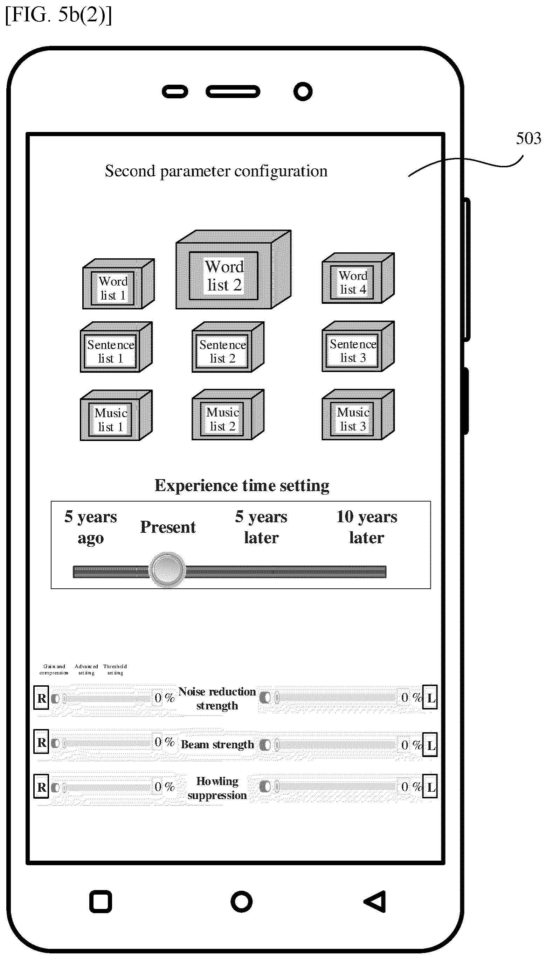

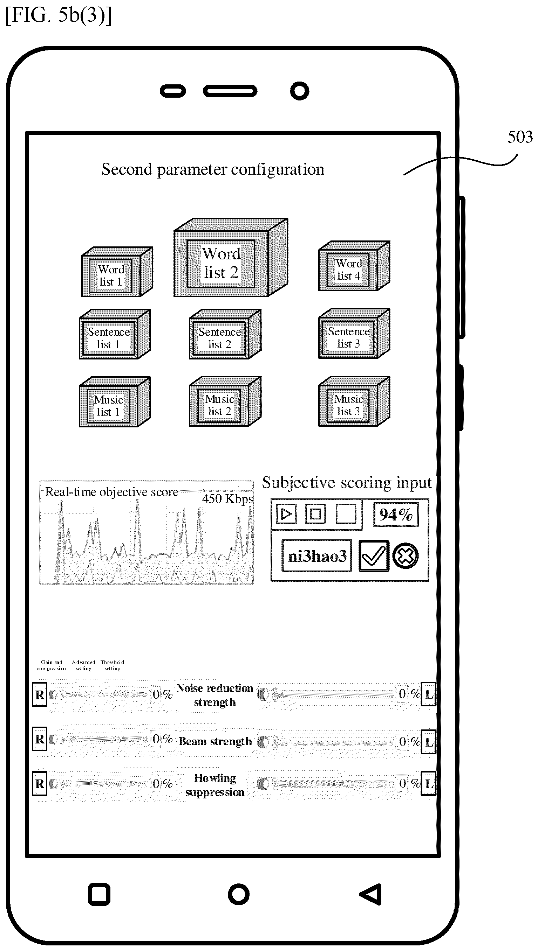

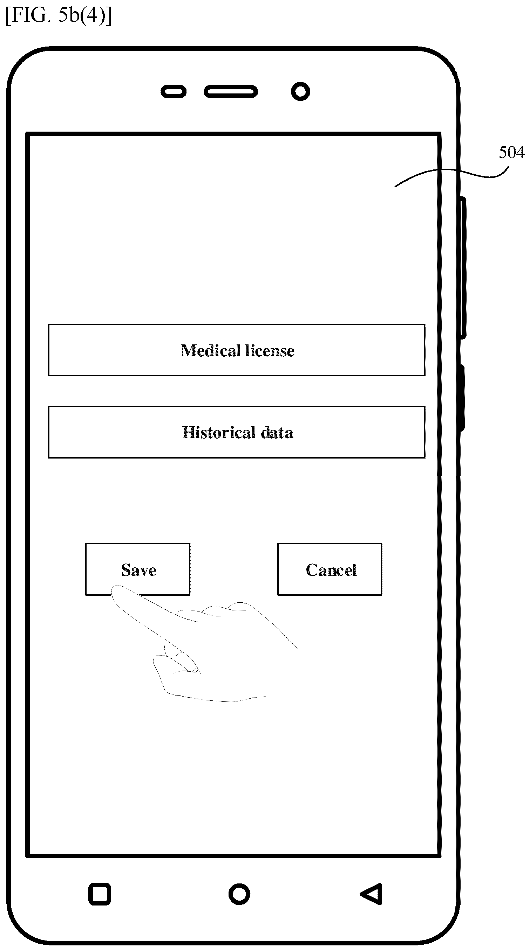

FIG. 5b(1) to FIG. 5b(4) are diagrams of examples of mobile phone interfaces.

With reference to FIG. 5b(1), for example, when the first user is a professional physician, the physician mode may be selected on the identity selection interface 501. After the user taps the physician mode option (that is, performs a second identity selection operation), the mobile phone may display a second parameter configuration interface 503 in response to the second identity selection operation, as shown in FIG. 5b(2). With reference to FIG. 5b(2), the second parameter configuration interface 503 may include one or more controls, including but not limited to: a scenario audio option (such as a word list 1 option, a word list 2 option, ..., a sentence list 1 option, a sentence list 2 option, ..., a music list 1 option, and a music list 2 option), an experience time setting option, a second parameter configuration item (such as a noise reduction strength configuration item, a beam strength configuration item, and a howling suppression configuration item), and the like. It should be understood that the second parameter configuration interface 503 may further include other options or other information (for example, the user information of the second user, such as the gender, the age, and the hearing loss duration). This is not limited in this application.

With reference to FIG. 5b(2), for example, on the second parameter configuration interface 503, the first user may first slide a slider knob in a sliding control corresponding to the experience time setting option to a scale mark. In this case, the mobile phone may obtain experience time information. Then one or more second parameter configuration items may be set (that is, a first parameter setting operation is performed). In this case, the mobile phone may obtain a first hearing aid algorithm parameter corresponding to a second identity. For a second parameter configuration item currently set by the first user, a first hearing aid algorithm parameter currently set by the first user may be obtained. For a second parameter configuration item currently not set by the first user, a first hearing aid algorithm parameter historically set by the first user or a default first hearing aid algorithm parameter may be obtained. The first hearing aid algorithm parameter corresponding to the second identity is a part of the second hearing aid algorithm parameter. Then the first user may select a scenario audio option (for example, the word list 2 option) from a plurality of scenario audio options (that is, perform a listening test operation). In this case, the mobile phone may send listening test data to the headset in response to the listening test operation, that is, perform S202. In this case, the listening test data may further include the first hearing aid algorithm parameter corresponding to the second identity. The first hearing aid algorithm parameter corresponding to the second identity may include but is not limited to noise reduction strength, beam strength, a degree of howling suppression, and the like. This is not limited in this application.

Then, after receiving the listening test data, the headset may obtain the first audio data, and when determining that the listening test data includes the first hearing aid algorithm parameter, the headset may start the hearing aid apparatus, and the hearing aid apparatus performs hearing aid optimization processing. Specifically, the hearing aid apparatus invokes the hearing aid algorithm to perform hearing aid optimization processing on the first audio data based on the first hearing aid algorithm parameter and other hearing aid algorithm parameters, so as to obtain fourth audio data and output the fourth audio data to the hearing loss simulation apparatus. Then the hearing loss simulation apparatus performs hearing loss simulation processing on the fourth audio data based on the hearing loss feature information, to obtain the second audio data. Refer to the foregoing description. Details are not described herein again.

For example, the terminal device may further display objective evaluation information in real time on the second parameter configuration interface 503, as shown in FIG. 5b(3). The objective evaluation information is determined based on an intelligibility score and a sound quality score, and in a real-time objective evaluation information box in 5b(3), a curve located above is determined based on the fourth audio data obtained through hearing aid optimization processing and output by the hearing aid apparatus, and a curve located below is determined based on the second audio data output by the hearing loss simulation apparatus (that is, determined based on the second evaluation information). In addition to the second intelligibility score and the second sound quality score, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

For example, the terminal device may further display a subjective scoring input item, and the first user may perform subjective scoring in the subjective scoring input item. For example, after hearing audio of a scenario audio option (for example, the word list 2 option) by using the headset, the first user may input a full spelling and a pitch of each word in the subjective scoring input item. After receiving information input by the first user in the subjective scoring input item, the terminal device collects statistics on accuracy, and uses the accuracy as a subjective score.

In this way, with reference to the foregoing manner, the first user may perform different configurations for the second parameter configuration item on the second parameter configuration interface 503 in FFIG. 5b(2), to experience audio data heard by the second user after the second user wears hearing aids with different hearing aid algorithm parameters; and select an optimal first hearing aid algorithm parameter with reference to subjective perception and objective evaluation information (which may include the second evaluation information), to configure an appropriate hearing aid algorithm parameter for the first user.

In a possible manner, the headset may be used as a hearing aid. After determining the first hearing aid algorithm parameter with an optimal effect, the first user may configure the second parameter configuration item on the second parameter configuration interface 503 in FIG. 5b(2) based on the optimal first hearing aid algorithm parameter. In this way, in a subsequent process in which the second user uses the headset as a hearing aid, the hearing aid algorithm of the hearing aid apparatus in the headset may perform hearing aid optimization processing by using the first hearing aid algorithm parameter with the optimal effect and other hearing aid algorithm parameters, and send the fourth audio data obtained through the hearing aid optimization processing to the speaker for playing (in this case, the hearing loss simulation apparatus does not run).

In a possible manner, after determining the first hearing aid algorithm parameter with the optimal effect, the first user may buy a hearing aid based on the first hearing aid algorithm parameter with the optimal effect.

Optionally, when the first user taps the physician mode option on the identity selection interface 501, the mobile phone may display an information input interface 504 in response to an operation of the first user, as shown in FIG. 5b(4). With reference to FIG. 5b(4), for example, the information input interface 504 may include one or more controls, including but not limited to a medical license option, a historical data option, a save option, a cancel option, and the like. This is not limited in this application.

With reference to FIG. 5b(4), for example, the first user may tap a medical license option on the information input interface 504 to upload the medical license. In this case, the mobile phone may obtain the medical license. For example, the first user may tap the historical data option on the information input interface 504, and upload historical user information (such as a historical audiogram or historical intelligibility test data) of the second user. In this case, the mobile phone may obtain the historical user information of the second user. The historical user information of the second user may be used as a reference for the physician to configure the second parameter configuration item. After determining that the medical license is successfully verified, the mobile phone may display information prompting to save the information. In this case, the first user may tap the save option on the information input interface 504, and the mobile phone may display the second parameter configuration interface 503 in response to an operation of the first user, as shown in FIG. 5b(2).

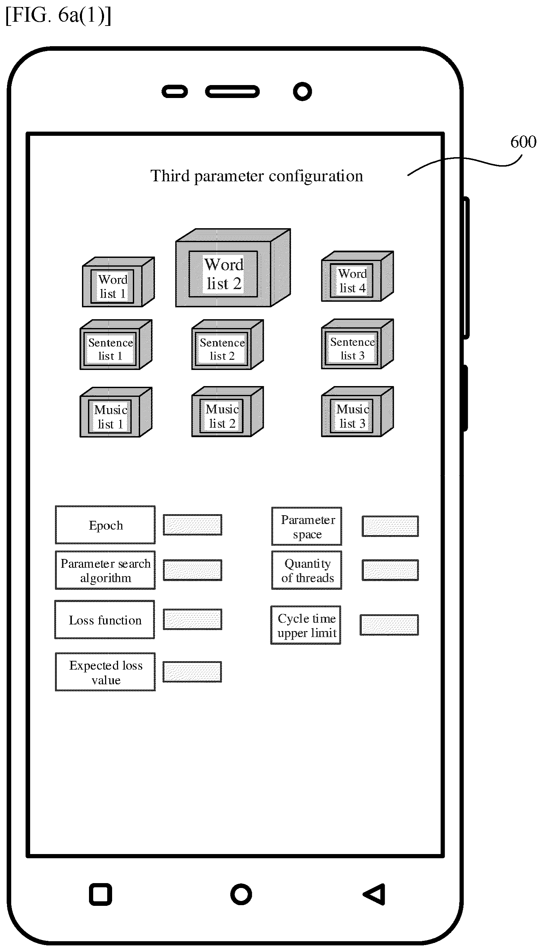

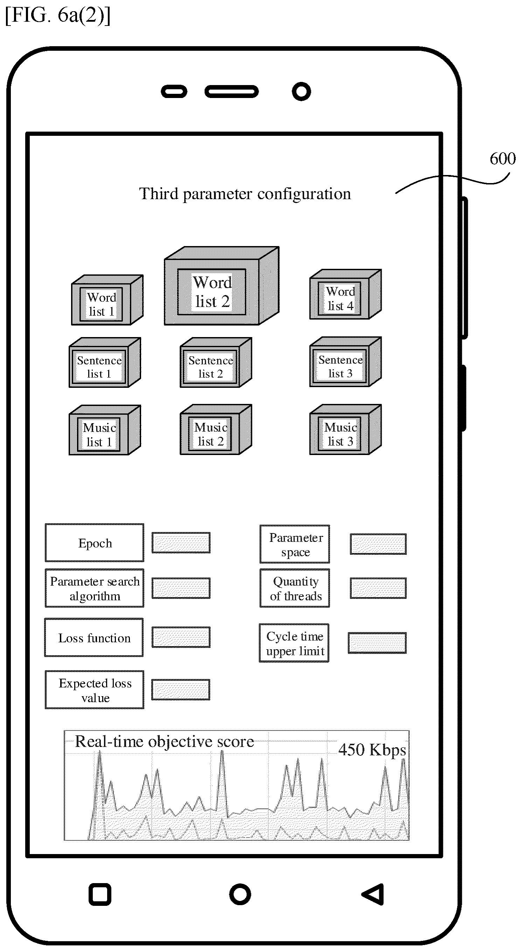

FIG. 6a(1) and FIG. 6a(2) are diagrams of examples of mobile phone interfaces.

With reference to FIG. 3a(2) again, for example, the first user may tap the second fitting option, and the hearing loss simulation system automatically fits the hearing aid algorithm parameter for the second user. For example, after the first user taps the second fitting option (that is, performs a second fitting operation), the mobile phone may receive the second fitting operation, and display a third parameter fitting interface 600, as shown in FIG. 6a(1). With reference to FIG. 6a(1), the third parameter configuration interface 600 may include one or more controls, including but not limited to: a scenario audio option (for example, a word list 1 option, a word list 2 option, ..., a sentence list 1 option, a sentence list 2 option, ..., a music list 1 option, and a music list 2 option), and a third parameter configuration item (a total epoch (epoch) quantity configuration item, a parameter search algorithm configuration item, a parameter space configuration item, a thread quantity configuration item, a loss (loss) function configuration item, an expected loss value configuration item, a cycle time upper limit configuration item, and the like). It should be understood that the third parameter configuration interface 600 may further include other options or other information (for example, the user information of the second user, such as the gender, the age, and the hearing loss duration). This is not limited in this application.

An epoch is a search (cycle/iteration) period. A parameter search algorithm may include but is not limited to a TPE (Tree-structured Parzen Estimator, a method for learning a hyperparameter model by using a Gaussian mixture model) algorithm, a random search (random search) algorithm, an annealing (Anneal) algorithm, a naive evolution (Naive evolution) algorithm, and the like. This is not limited in this application. A parameter search space is a range of a hearing aid algorithm parameter, for example, the hearing aid algorithm gain parameter ∈ [-10 dB, 10 dB]. A quantity of parallel threads is a quantity of parallel threads used during a hearing aid algorithm parameter search cycle or iteration.

With reference to FIG. 6a(1), for example, on the third parameter configuration interface 600, one or more third parameter configuration items may be set (that is, a second parameter setting operation is performed). In this case, the mobile phone may obtain an optimization parameter. For a third parameter configuration item currently set by the first user, an optimization parameter currently set by the first user may be obtained. For a third parameter configuration item currently not set by the first user, an optimization parameter historically set by the first user or a default optimization parameter may be obtained. The optimization parameter is a parameter used to search for the second hearing aid algorithm parameter. Then the first user may select a scenario audio option (for example, the word list 2 option) from a plurality of scenario audio options (that is, perform a listening test operation). In this case, the mobile phone may send listening test data to the headset in response to the listening test operation, that is, perform S202. In this case, the listening test data may further include the optimization parameter. The optimization parameter may include but is not limited to: an epoch quantity, a parameter search algorithm, a loss function, a parameter search space, a parallel thread quantity, an expected loss value, a cycle time upper limit, and the like. This is not limited in this application.

Then, after receiving the listening test data, the headset may obtain the first audio data, and when it is determined that the listening test data includes the optimization parameter, search for the second hearing aid algorithm parameter based on the optimization parameter. Then the hearing aid algorithm of the hearing aid apparatus is enabled. The hearing aid apparatus invokes the hearing aid algorithm, and performs hearing aid optimization processing on the first audio data based on the second hearing aid algorithm parameter obtained through the search, to obtain fifth audio data and output the fifth audio data to the hearing loss simulation apparatus. Then the hearing loss simulation apparatus performs hearing loss simulation processing on the fifth audio data based on the hearing loss feature information, to obtain the second audio data. Refer to the foregoing description. Details are not described herein again.

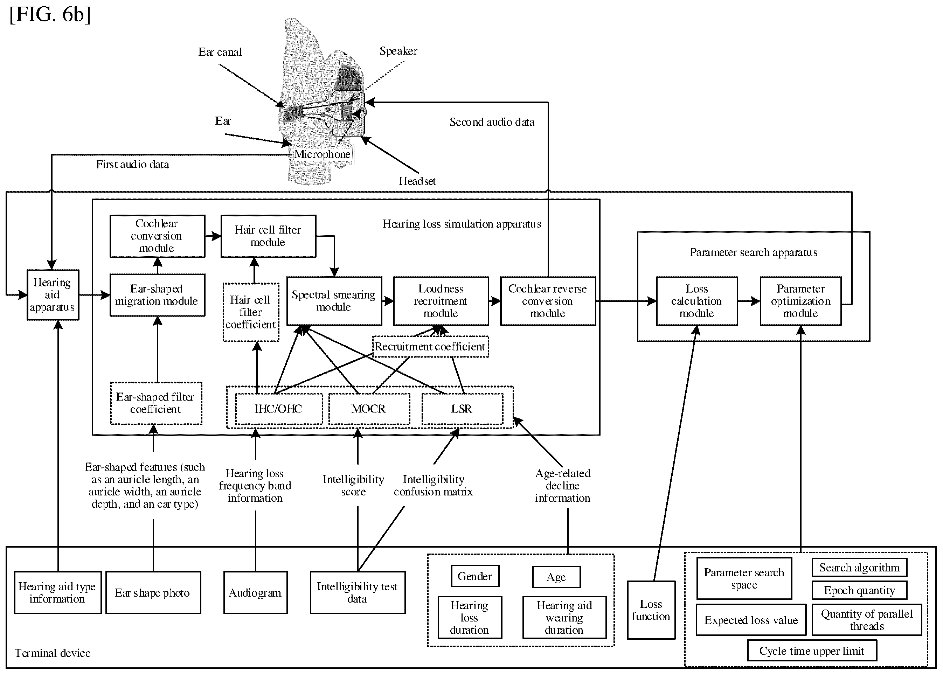

FIG. 6b is a diagram of an example of a hearing loss simulation system. FIG. 6b is shown on a basis of FIG. 4b. A parameter search apparatus is located in the headset, and the parameter search apparatus may be implemented by a DSP in the headset. The parameter search apparatus may include a loss calculation module and a parameter optimization module. The loss calculation module may calculate a loss value based on a loss function. The parameter optimization module may be configured to optimize a hearing aid algorithm parameter based on the loss value.

With reference to FIG. 6b, for example, after obtaining the first audio data, the headset may input the first audio data to the hearing aid apparatus, so that the hearing aid apparatus invokes the hearing aid algorithm to perform hearing aid optimization on the first audio data based on an initial hearing aid algorithm parameter, to obtain the first audio data on which hearing aid optimization processing is performed, and output the first audio data to the hearing loss simulation apparatus. Then the hearing loss simulation apparatus performs hearing loss simulation based on the hearing loss feature information, and outputs the second audio data to the loss calculation module. Then the loss calculation module calculates the second audio data by using the loss function included in the listening test data, to obtain a loss value, and outputs the loss value to the parameter optimization module. Then the parameter optimization module may use the loss value as prior knowledge to guide a direction of parameter optimization in a next iteration, so as to generate an optimized hearing aid algorithm parameter. Specifically, the parameter optimization module may analyze a loss value under an effect of a previous hearing aid algorithm parameter by using the parameter search algorithm, guide the direction of the next parameter optimization, optimize the previous hearing aid algorithm parameter based on the optimization direction, and determine an optimized hearing aid algorithm parameter, thereby achieving an effect that the loss value is closer to an expected loss value included in the listening test data.

Then the hearing aid apparatus invokes the hearing aid algorithm to perform hearing aid optimization on the first audio data by using the optimized hearing aid algorithm parameter, and outputs the first audio data on which hearing aid optimization processing is performed to the hearing loss simulation apparatus. The hearing loss simulation apparatus performs hearing loss simulation and outputs the second audio data to the loss calculation module. The loss calculation module calculates the loss value and outputs the loss value to the parameter optimization module, and the parameter optimization module performs parameter optimization again to obtain an optimized hearing aid algorithm parameter, and so on, until the parameter optimization module determines that a quantity of cycles reaches the epoch quantity included in the listening test data, or a latest loss value has been optimized and reached the expected loss value included in the listening test data, or a program runtime or cycle time reaches the cycle time upper limit included in the listening test data. In this case, the hearing aid algorithm parameter obtained through optimization is a final hearing aid algorithm parameter (that is, the second hearing aid algorithm parameter).

Then the hearing aid apparatus invokes the hearing aid algorithm to perform hearing aid optimization processing on the first audio data by using the foregoing determined final hearing aid algorithm parameter, to obtain fifth audio data. Then the hearing loss simulation apparatus performs hearing loss simulation on the fifth audio data based on the hearing loss feature information, to obtain the second audio data and outputs the second audio data to the speaker for playing.

For example, the terminal device may further display objective evaluation information in real time on the third parameter configuration interface 600, as shown by a curve in a real-time objective score box in FIG. 6a(2). The objective evaluation information is determined based on an intelligibility score and a sound quality score, and in the real-time objective score box in FIG. 6a(2), a curve located above is determined based on the fifth audio data output by the hearing aid apparatus, and a curve located below is determined based on the second audio data output by the hearing loss simulation apparatus (that is, determined based on the second evaluation information). In addition to the second intelligibility score and the second sound quality score, the second evaluation information may further include information obtained by testing the second audio data based on related requirements for the effect (or function or performance) of the hearing aid in related standards (for example, international standards for hearing aids, national standards for hearing aids, industry standards for hearing aids, and community standards for hearing aids). For example, the second evaluation information may further include a noise suppression capability, an anti-howling capability, a frequency response range, a real-ear insertion gain, a maximum output sound gain, and the like. This is not limited in this application.

In this way, with reference to the foregoing manner, the first user may perform different configurations for the third parameter configuration item on the third parameter configuration interface 600 in FIG. 6a(1), to experience audio data heard by the second user after the second user wears hearing aids with different second hearing aid algorithm parameters; and select an optimal optimization parameter with reference to subjective perception and objective evaluation information (which may include the second evaluation information), to configure an appropriate second hearing aid algorithm parameter for the first user.

In a possible manner, the headset may be used as a hearing aid. After determining the optimization parameter with an optimal effect, the first user may configure the third parameter configuration item on the third parameter configuration interface 600 in FIG. 6a(1) based on the optimal optimization parameter. In this way, in a subsequent process in which the second user uses the headset as a hearing aid, the hearing aid algorithm of the hearing aid apparatus in the headset may perform hearing aid optimization processing by using the second hearing aid algorithm parameter with an optimal effect, and send the fifth audio data obtained through the hearing aid optimization processing to the speaker for playing (in this case, the hearing loss simulation apparatus does not run).

In a possible manner, after determining the optimization parameter with the optimal effect, the first user may view, from the terminal device, the second hearing aid algorithm parameter with the optimal effect; and then may buy a hearing aid based on the second hearing aid algorithm parameter with the optimal effect.

Optionally, after the first user taps the second fitting option (that is, performs the second fitting operation), the mobile phone may receive the second fitting operation, and display the information input interface 504, as shown in FIG. 5b(4). With reference to FIG. 5b(4), for example, the first user may tap the medical license option on the information input interface 504 to upload the medical license. In this case, the mobile phone may obtain the medical license. For example, the first user may tap the historical data option on the information input interface 504, and upload the historical first hearing aid algorithm parameter of the second user. The historical first hearing aid algorithm parameter of the second user may be used as an initial value in a process of searching for the second hearing aid algorithm parameter. After determining that the medical license is successfully verified, the mobile phone may display information prompting to save the information. In this case, the first user may tap the save option on the information input interface 504, and the mobile phone may display the third parameter configuration interface 600 in response to the operation of the first user, as shown in FIG. 6a(1).

It should be noted that, with reference to FIG. a(1), in a possible manner, after the first user taps the application icon 302 of the hearing loss simulation application, the mobile phone displays the function selection interface 304 in response to the operation of the first user, as shown in FIG. 3a(3). Subsequently, when the first user taps any one of the hearing loss effect experience option, the hearing aid effect experience option, the first fitting option, and the second fitting option on the function selection interface 304, the mobile phone may display the information input interface 303 in response to an operation of the first user, as shown in FIG. 3a(2). In other words, this application does not limit whether the mobile phone displays the function selection interface 304 or displays the information input interface 303 after the first user taps the application icon 302 of the hearing loss simulation application.

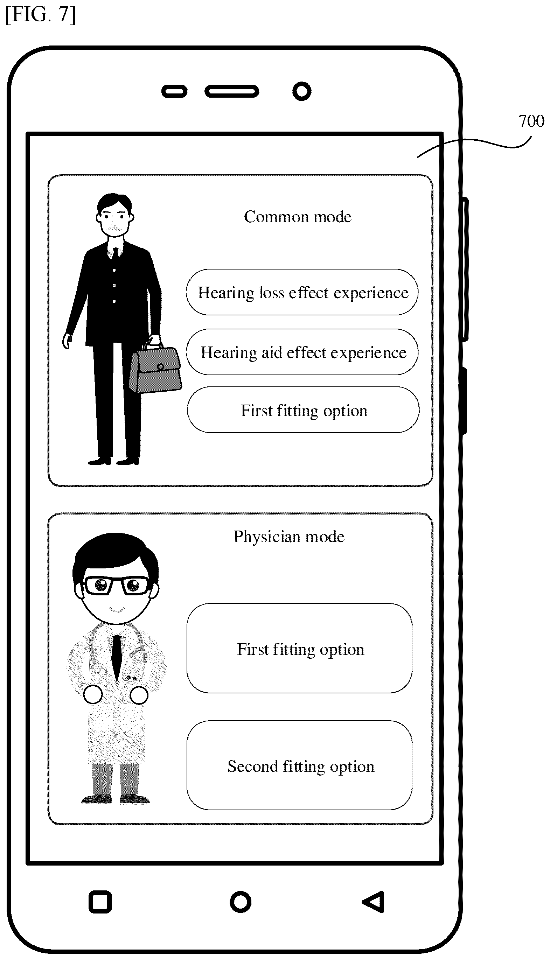

It should be noted that, with reference to FIG. 3a(1), in a possible manner, the first user taps the application icon 302 of the hearing loss simulation application, and the mobile phone displays a function selection interface 700 in response to an operation of the first user, as shown in FIG. 7. With reference to FIG. 7, for example, the function selection interface 700 includes one or more controls, including but not limited to: a plurality of function options (such as a hearing loss effect experience option, a hearing aid effect experience option, and a first fitting option) located in a common mode selection area, and a plurality of function options (such as a first fitting option and a second fitting option) located in a physician mode selection area. It should be understood that the function selection interface 700 may further include other options or information. This is not limited in this application.

With reference to FIG. 7, for example, when the first user taps any one of the hearing loss effect experience option, the hearing aid effect experience option, the first fitting option, and the second fitting option on the function selection interface 700, the mobile phone may display the information input interface 303 in response to an operation of the first user, as shown in FIG. 3a(2). In this case, after the first user taps the first fitting option (that is, performs a first fitting operation) in the common mode selection area in FIG. 7, the mobile phone may receive the first fitting operation. Then the mobile phone may display the first parameter configuration interface 502 in response to the first fitting operation, as shown in 5a(2). After the first user taps the first fitting option (that is, performs a first fitting operation) in the physician mode selection area in FIG. 7, the mobile phone may receive the first fitting operation. Then the mobile phone may display the second parameter configuration interface 503 in response to the first fitting operation, as shown in 5b(2).

The following uses the hearing loss simulation system in FIG. 1c as an example to describe a process in which a user without hearing loss experiences an effect of audio heard by a user with hearing loss. For ease of subsequent description, the user without hearing loss is referred to as a first user, and the user with hearing loss is referred to as a second user. In addition, the headset is worn by the first user.

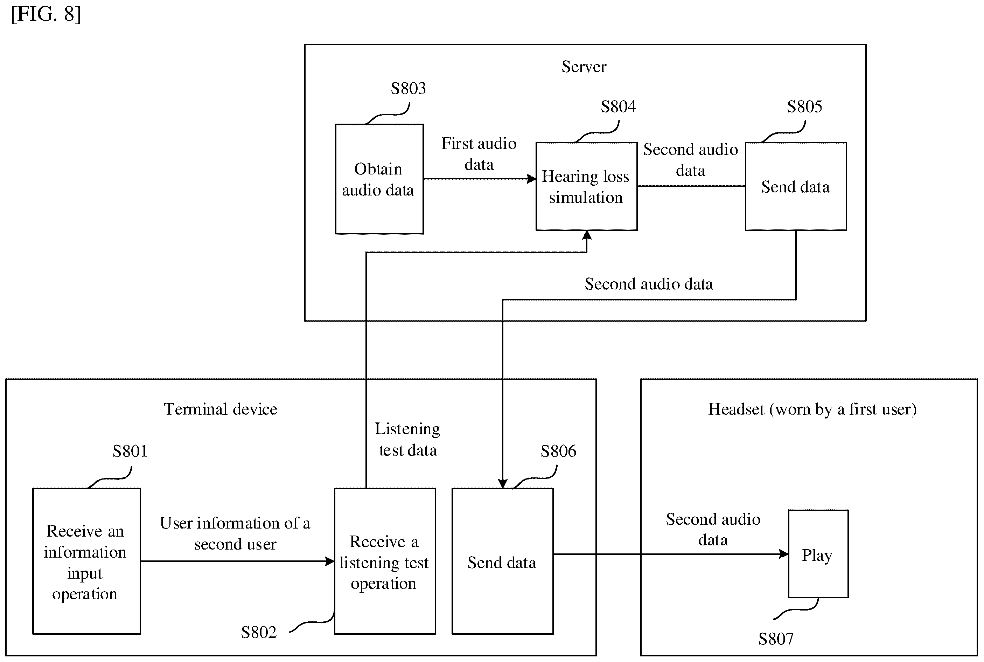

FIG. 8 is a diagram of an example of a hearing loss experience process.

S801: A terminal device receives an information input operation, and obtains user information of a second user that is correspondingly input by the information input operation.

S802: The terminal device receives a listening test operation, and sends listening test data to a server in response to the listening test operation, where the listening test data includes the user information or hearing loss feature information, and the hearing loss feature information is determined based on the user information.

S803: The server obtains first audio data.

In a possible manner, the terminal device may send preset audio data corresponding to a scenario audio option corresponding to the listening test operation to the server as the first audio data.

In a possible manner, the terminal device may send an audio collection start indication to a headset, obtain the first audio data from the headset, and send the first audio data to the server.

In a possible manner, the listening test data may include a scenario audio identifier corresponding to the scenario audio option corresponding to the listening test operation. In this way, the server may obtain, from prestored preset audio data, preset audio data corresponding to the scenario audio identifier as the first audio data.

S804: The server performs hearing loss simulation on the first audio data based on the hearing loss feature information, to obtain second audio data.

For example, for S804, refer to the descriptions of S204. Details are not described herein again.

S805: The server sends the second audio data to the terminal device.

S806: The terminal device sends the second audio data to the headset.

S807: The headset plays the second audio data.

For example, for descriptions of S801 to S807, refer to the foregoing descriptions. Details are not described herein again.

For example, on a basis of the embodiment in FIG. 8, the first user may further select an appropriate hearing aid type for the second user, and configure an appropriate hearing aid algorithm parameter. For details, refer to the foregoing descriptions. Details are not described herein again. In this case, the hearing loss simulation apparatuses in FIG. 3b, FIG. 4b, and FIG. 6b are located on the server, the hearing aid apparatuses in FIG. 4b and FIG. 6b are located on the server, and the parameter search apparatus in FIG. 6b is located on the server.

The following uses the hearing loss simulation system in FIG. 1d as an example to describe a process in which a user without hearing loss experiences an effect of audio heard by a user with hearing loss. For ease of subsequent description, the user without hearing loss is referred to as a first user, and the user with hearing loss is referred to as a second user. In addition, the headset is worn by the first user.

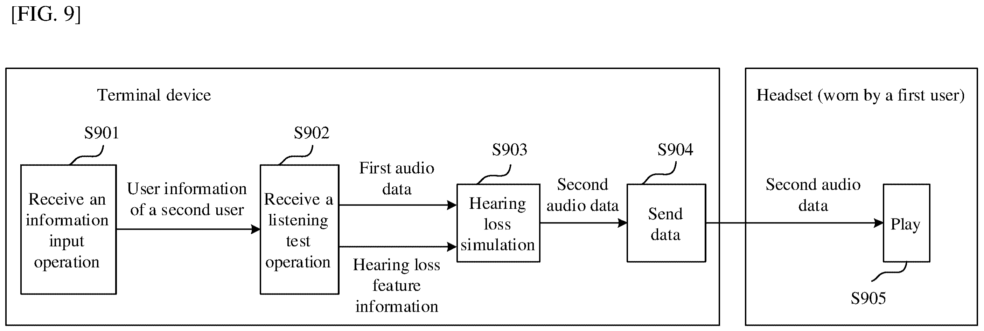

FIG. 9 is a diagram of an example of a hearing loss experience process.

S901: A terminal device receives an information input operation, and obtains user information of a second user that is correspondingly input by the information input operation.

S902: The terminal device receives a listening test operation, obtains first audio data in response to the listening test operation, and generates hearing loss feature information based on the user information.

In a possible manner, the terminal device may use preset audio data corresponding to a scenario audio option corresponding to the listening test operation as the first audio data.

In a possible manner, the terminal device may send an audio collection start indication to a headset, and obtain the first audio data from the headset.

S903: The terminal device performs hearing loss simulation on the first audio data based on the hearing loss feature information, to obtain second audio data.

S904: The terminal device sends the second audio data to the headset.

S905: The headset plays the second audio data.

For example, for descriptions of S901 to S905, refer to the foregoing descriptions. Details are not described herein again.

For example, on a basis of the embodiment in FIG. 9, the first user may further select an appropriate hearing aid type for the second user, and configure an appropriate hearing aid algorithm parameter. For details, refer to the foregoing descriptions. Details are not described herein again. In this case, the hearing loss simulation apparatuses in FIG. 3b, FIG. 4b, and FIG. 6b are located on the terminal device, the hearing aid apparatuses in FIG. 4b and FIG. 6b are located on the terminal device, and the parameter search apparatus in FIG. 6b is located on the terminal device.

It should be noted that evaluation information (which may include the first evaluation information, the second evaluation information, and the third evaluation information) may be calculated by the terminal device, the server, the headset, or another device. This is not limited in this application. For example, when the terminal device calculates the evaluation information, the terminal device may display the evaluation information, or may transmit the evaluation information to another terminal device to display the evaluation information. When the headset/server calculates the evaluation information, the headset/server may transmit the evaluation information to the terminal device or another terminal device to display the evaluation information. In addition, after calculating the evaluation information to obtain the evaluation information through calculation, the terminal device, the server, the headset, or the another device may store the evaluation information for subsequent viewing and use. In other words, a type of an electronic device for calculating the evaluation information is not limited in this application, and a manner of using the evaluation information is not limited either.

It should be further noted that the three processing operations of hearing loss simulation, hearing aid optimization, and searching for the second hearing aid algorithm parameter may be decoupled. In other words, two or more processing operations of hearing loss simulation, hearing aid optimization, and searching for the second hearing aid algorithm parameter may be performed by a same device, or may be performed by different devices. This is not limited in this application.

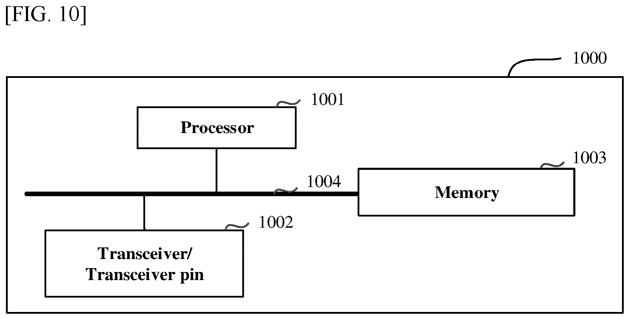

In an example, FIG. 10 is a block diagram of an apparatus 1000 according to an embodiment of this application. The apparatus 1000 may include a processor 1001 and a transceiver or transceiver pin 1002, and optionally, further includes a memory 1003.

Components of the apparatus 1000 are coupled together through a bus 1004. In addition to a data bus, the bus 1004 further includes a power bus, a control bus, and a status signal bus. However, for clarity of description, various buses are referred to as the bus 1004 in the figure.

Optionally, the memory 1003 may be configured to store instructions in the foregoing method embodiments. The processor 1001 may be configured to execute the instructions in the memory 1003, control a receive pin to receive a signal, and control a transmit pin to send a signal.

The apparatus 1000 may be the electronic device in the foregoing method embodiments or a chip of the electronic device. The electronic device may be a headset, a terminal device, or a server.

All related content of steps in the foregoing method embodiments may be cited in function descriptions of corresponding functional modules. Details are not described herein again.

An embodiment further provides a chip. The chip includes one or more interface circuits and one or more processors. The interface circuit is configured to receive a signal from a memory of an electronic device, and send the signal to the processor. The signal includes computer instructions stored in the memory. When the processor executes the computer instructions, the electronic device is enabled to perform the method in the foregoing embodiments. The interface circuit may be the transceiver or transceiver pin 1002.

An embodiment further provides a computer-readable storage medium. The computer-readable storage medium stores computer instructions. When the computer instructions are run on an electronic device, the electronic device is enabled to perform the foregoing related method steps, to implement the steps in the foregoing embodiments.

An embodiment further provides a computer program product. The computer program product includes computer instructions. When the computer instructions are executed by a computer or a processor, the computer is enabled to perform the foregoing related steps, to implement the steps in the foregoing embodiments.

In addition, an embodiment of this application further provides an apparatus. The apparatus may be specifically a chip, a component, or a module. The apparatus may include a processor and a memory that are connected to each other. The memory is configured to store computer-executable instructions. When the apparatus runs, the processor may execute the computer-executable instructions stored in the memory, so that the chip performs the steps in the foregoing method embodiments.

The electronic device, the computer-readable storage medium, the computer program product, or the chip provided in the embodiments is configured to perform the corresponding method provided above. Therefore, for beneficial effects that can be achieved, refer to the beneficial effects in the corresponding method provided above. Details are not described herein again.

Based on the descriptions of the foregoing implementations, a person skilled in the art may understand that, for ease and brevity of description, only division into the foregoing functional modules is used as an example for description; in actual application, the foregoing functions may be allocated to different functional modules and implemented based on requirements. In other words, an inner structure of an apparatus is divided into different functional modules to implement all or some of the functions described above.

In the several embodiments provided in this application, it should be understood that the disclosed apparatus and method may be implemented in other manners. For example, the described apparatus embodiment is merely an example. For example, division into the modules or units is merely logical function division and may be other division in actual implementation. For example, a plurality of units or components may be combined, or may be integrated into another apparatus, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

Units described as separate components may or may not be physically separate. A component displayed as a unit may be one or more physical units, and may be located in one place, or may be distributed in a plurality of different places. Some or all of the units may be selected depending on actual requirements, to achieve the objectives of the solutions in embodiments.

In addition, functional units in embodiments of this application may be integrated into one processing unit, each of the units may exist alone physically, or two or more units are integrated into one unit. The integrated unit may be implemented in a form of hardware, or may be implemented in a form of a software functional unit.

Any content in embodiments of this application and any content in a same embodiment can be freely combined. Any combination of the foregoing content falls within the scope of this application.

When the integrated unit is implemented in the form of a software functional unit and is sold or used as an independent product, the integrated unit may be stored in a readable storage medium. Based on such an understanding, the technical solutions in embodiments of this application essentially, or the part contributing to the conventional technology, or all or some of the technical solutions may be implemented in a form of a software product. The software product is stored in a storage medium, and includes several instructions for instructing a device (which may be a single-chip microcomputer, a chip, or the like) or a processor (processor) to perform all or some of the steps of the method described in embodiments of this application. The storage medium includes any medium that can store program code, such as a USB flash drive, a removable hard disk, a read-only memory (read-only memory, ROM), a random access memory (random access memory, RAM), a magnetic disk, or an optical disc.

The foregoing describes embodiments of this application with reference to the accompanying drawings. However, this application is not limited to the foregoing specific embodiments. The foregoing specific embodiments are merely illustrative rather than restrictive. Inspired by this application, a person of ordinary skill in the art may develop many other manners without departing from principles of this application and the protection scope of the claims, and all such manners fall within the protection scope of this application.

Method or algorithm steps described in combination with content disclosed in embodiments of this application may be implemented by hardware, or may be implemented by a processor by executing software instructions. The software instructions may include a corresponding software module. The software module may be stored in a random access memory (Random Access Memory, RAM), a flash memory, a read only memory (Read Only Memory, ROM), an erasable programmable read only memory (Erasable Programmable ROM, EPROM), an electrically erasable programmable read only memory (Electrically EPROM, EEPROM), a register, a hard disk, a removable hard disk, a compact disc read-only memory (CD-ROM), or any other form of storage medium well-known in the art. For example, the storage medium is coupled to the processor, so that the processor can read information from the storage medium or write information into the storage medium. Certainly, the storage medium may alternatively be a component of the processor. The processor and the storage medium may be located in an ASIC.

A person skilled in the art should be aware that in one or more of the foregoing examples, functions described in embodiments of this application may be implemented by using hardware, software, firmware, or any combination thereof. When the functions are implemented by software, the functions may be stored in a computer-readable medium or transmitted as one or more instructions or code on a computer-readable medium. The computer-readable medium includes a computer-readable storage medium and a communication medium, where the communication medium includes any medium that enables a computer program to be transmitted from one place to another. The storage medium may be any available medium accessible to a general-purpose or dedicated computer.

The foregoing describes embodiments of this application with reference to the accompanying drawings. However, this application is not limited to the foregoing specific embodiments. The foregoing specific embodiments are merely illustrative rather than restrictive. Inspired by this application, a person of ordinary skill in the art may develop many other manners without departing from principles of this application and the protection scope of the claims, and all such manners fall within the protection scope of this application.

## Claims

1. A hearing loss simulation system, wherein the hearing loss simulation system comprises a headset and a terminal device, and the headset is worn by a first user, wherein
the terminal device is configured to: receive an information input operation, and obtain user information of a second user that is correspondingly input by the information input operation; and receive a listening test operation, and send listening test data to the headset in response to the listening test operation, wherein the listening test data comprises the user information or hearing loss feature information, and the hearing loss feature information is determined based on the user information; and
the headset is configured to: obtain first audio data; perform hearing loss simulation on the first audio data based on the hearing loss feature information, to obtain second audio data; and play the second audio data.

2. The system according to claim 1, wherein
the terminal device is further configured to display first evaluation information of the first audio data and second evaluation information of the second audio data.

3. The system according to claim 1 or 2, wherein
the terminal device is further configured to display text information corresponding to the first audio data, and annotate a target word in the text information based on the second audio data, wherein
an intelligibility score of the target word is less than an intelligibility threshold, and/or the target word is a word whose sound quality score is less than a sound quality threshold.

4. The system according to any one of claims 1 to 3, wherein
before receiving the listening test operation, the terminal device is further configured to receive an experience time setting operation, and obtain experience time information that is correspondingly set by the experience time setting operation, wherein
the listening test data further comprises the experience time information, and the hearing loss feature information is determined based on the user information and the experience time information.

5. The system according to any one of claims 1 to 4, wherein
before receiving the listening test operation, the terminal device is further configured to receive a hearing aid type selection operation, and obtain hearing aid type information corresponding to the hearing aid type selection operation, wherein the listening test data further comprises the hearing aid type information; and
the headset is further configured to: perform hearing aid optimization processing on the first audio data by using a hearing aid algorithm corresponding to the hearing aid type information, to obtain third audio data; and perform hearing loss simulation processing on the third audio data based on the hearing loss feature information, to obtain the second audio data.

6. The system according to any one of claims 1 to 4, wherein
before receiving the listening test operation, the terminal device is further configured to receive a first parameter setting operation, and obtain a first hearing aid algorithm parameter corresponding to the first parameter setting operation, wherein the listening test data further comprises the first hearing aid algorithm parameter; and
the headset is further configured to: perform hearing aid optimization processing on the first audio data based on the first hearing aid algorithm parameter, to obtain fourth audio data; and perform hearing loss simulation processing on the fourth audio data based on the hearing loss feature information, to obtain the second audio data.

7. The system according to any one of claims 1 to 4, wherein
before receiving the listening test operation, the terminal device is further configured to receive a second parameter setting operation, and obtain an optimization parameter corresponding to the second parameter setting operation, wherein the listening test data further comprises the optimization parameter; and the headset is further configured to: search for a second hearing aid algorithm parameter based on the optimization parameter; perform hearing aid optimization processing on the first audio data based on the second hearing aid algorithm parameter, to obtain fifth audio data; and perform hearing loss simulation processing on the fifth audio data based on the hearing loss feature information, to obtain the second audio data.

8. The system according to any one of claims 5 to 7, wherein
the headset is further configured to: perform hearing loss simulation on the first audio data based on the hearing loss feature information, to obtain sixth audio data; and play the sixth audio data; and
the terminal device is further configured to display third evaluation information of the sixth audio data.

9. The system according to any one of claims 1 to 8, wherein
the headset is specifically configured to: determine a hearing loss simulation parameter based on the hearing loss feature information, wherein the hearing loss simulation parameter comprises a hair cell filter coefficient; and perform hair cell filtering processing on the first audio data based on the hair cell filter coefficient, to obtain the second audio data.

10. The system according to claim 9, wherein the hearing loss simulation parameter
further comprises an ear-shaped filter coefficient and a recruitment coefficient; and
the headset is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

11. A hearing loss simulation system, wherein the hearing loss simulation system comprises a headset, a terminal device, and a server, and the headset is worn by a first user, wherein
the terminal device is configured to: receive an information input operation, and obtain user information of a second user that is correspondingly input by the information input operation; and receive a listening test operation, and send listening test data to the server in response to the listening test operation, wherein the listening test data comprises the user information or hearing loss feature information, and the hearing loss feature information is determined based on the user information;
the server is configured to: obtain first audio data; perform hearing loss simulation processing on the first audio data based on the hearing loss feature information, to obtain second audio data; and send the second audio data to the terminal device;
the terminal device is further configured to send the second audio data to the headset; and
the headset is configured to play the second audio data.

12. The system according to claim 11, wherein
the terminal device is further configured to display first evaluation information of the first audio data and second evaluation information of the second audio data.

13. The system according to claim 11 or 12, wherein
the terminal device is further configured to display text information corresponding to the first audio data, and annotate a target word in the text information based on the second audio data, wherein
an intelligibility score of the target word is less than an intelligibility threshold, and/or the target word is a word whose sound quality score is less than a sound quality threshold.

14. The system according to any one of claims 11 to 13, wherein
before receiving the listening test operation, the terminal device is further configured to receive an experience time setting operation, and obtain experience time information that is correspondingly set by the experience time setting operation, wherein
the listening test data further comprises the experience time information, and the hearing loss feature information is determined based on the user information and the experience time information.

15. The system according to any one of claims 11 to 14, wherein
before receiving the listening test operation, the terminal device is further configured to receive a hearing aid type selection operation, and obtain hearing aid type information corresponding to the hearing aid type selection operation, wherein the listening test data further comprises the hearing aid type information; and
the server is further configured to: perform hearing aid optimization processing on the first audio data by using a hearing aid algorithm corresponding to the hearing aid type information, to obtain third audio data; and perform hearing loss simulation processing on the third audio data based on the hearing loss feature information, to obtain the second audio data.

16. The system according to any one of claims 11 to 14, wherein
before receiving the listening test operation, the terminal device is further configured to receive a first parameter setting operation, and obtain a first hearing aid algorithm parameter corresponding to the first parameter setting operation, wherein the listening test data further comprises the first hearing aid algorithm parameter; and
the server is further configured to: perform hearing aid optimization processing on the first audio data based on the first hearing aid algorithm parameter, to obtain fourth audio data; and perform hearing loss simulation processing on the fourth audio data based on the hearing loss feature information, to obtain the second audio data.

17. The system according to any one of claims 11 to 14, wherein
before receiving the listening test operation, the terminal device is further configured to receive a second parameter setting operation, and obtain an optimization parameter corresponding to the second parameter setting operation, wherein the listening test data further comprises the optimization parameter; and the server is further configured to: search for a second hearing aid algorithm parameter based on the optimization parameter; perform hearing aid optimization processing on the first audio data based on the second hearing aid algorithm parameter, to obtain fifth audio data; and perform hearing loss simulation processing on the fifth audio data based on the hearing loss feature information, to obtain the second audio data.

18. The system according to any one of claims 15 to 17, wherein
the server is further configured to: perform hearing loss simulation on the first audio data based on the hearing loss feature information, to obtain sixth audio data; and send the sixth audio data to the terminal device;
the terminal device is further configured to send the sixth audio data to the headset;
the headset is further configured to play the sixth audio data; and
the terminal device is further configured to display third evaluation information of the sixth audio data.

19. The system according to any one of claims 11 to 18, wherein
the server is specifically configured to: determine a hearing loss simulation parameter based on the hearing loss feature information, wherein the hearing loss simulation parameter comprises a hair cell filter coefficient; and perform hair cell filtering processing on the first audio data based on the hair cell filter coefficient, to obtain the second audio data.

20. The system according to claim 19, wherein the hearing loss feature information
further comprises an ear-shaped filter coefficient and a recruitment coefficient; and
the server is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

21. A hearing loss simulation system, wherein the hearing loss simulation system
comprises a headset and a terminal device, and the headset is worn by a first user, wherein
the terminal device is configured to: receive an information input operation, and obtain user information of a second user that is correspondingly input by the information input operation; receive a listening test operation, obtain first audio data in response to the listening test operation, and generate hearing loss feature information based on the user information; perform hearing loss simulation on the first audio data based on the hearing loss feature information, to obtain second audio data; and send the second audio data to the headset; and
the headset is configured to play the second audio data.

22. A headset, wherein the headset is worn by a first user, and the headset is
configured to:
receive listening test data, wherein the listening test data comprises user information of a second user or hearing loss feature information, and the hearing loss feature information is determined based on the user information;
obtain first audio data;
determine a hearing loss simulation parameter based on the hearing loss feature information, wherein the hearing loss simulation parameter comprises a hair cell filter coefficient;
perform hair cell filtering processing on the first audio data based on the hair cell filter coefficient, to obtain second audio data; and
play the second audio data.

23. The headset according to claim 22, wherein the hearing loss simulation
parameter further comprises an ear-shaped filter coefficient and a recruitment coefficient; and
the headset is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

24. The headset according to claim 22 or 23, wherein
the headset is configured to: when the listening test data further comprises the first audio data, extract the first audio data from the listening test data; or when the listening test data further comprises an audio collection start indication, start an audio collection module to collect the first audio data.

25. The headset according to any one of claims 22 to 24, wherein
before performing hair cell filtering processing on the first audio data based on the hair cell filter coefficient, to obtain the second audio data, the headset is further configured to:
when the listening test data further comprises the hearing aid type information, perform hearing aid optimization processing on the first audio data by using a hearing aid algorithm corresponding to the hearing aid type information;
when the listening test data further comprises a first hearing aid algorithm parameter, perform hearing aid optimization processing on the first audio data based on the first hearing aid algorithm parameter; or
when the listening test data further comprises an optimization parameter, search for a second hearing aid algorithm parameter based on the optimization parameter; and perform hearing aid optimization processing on the first audio data based on the second hearing aid algorithm parameter.

26. The headset according to any one of claims 22 to 25, wherein
the hearing loss feature information comprises at least one of the following: an ear-shaped feature, hearing loss frequency band information, an intelligibility score, an intelligibility confusion matrix, or age-related decline information.

27. A server, wherein the server is configured to:
receive listening test data, wherein the listening test data comprises user information of a second user or hearing loss feature information, and the hearing loss feature information is determined based on the user information;
obtain first audio data;
determine a hearing loss simulation parameter based on the hearing loss feature information, wherein the hearing loss simulation parameter comprises a hair cell filter coefficient;
perform hair cell filtering processing on the first audio data based on the hair cell filter coefficient, to obtain second audio data; and
send the second audio data to a headset, so that the headset plays the second audio data, wherein the headset is worn by a first user.

28. The server according to claim 27, wherein the hearing loss simulation parameter
further comprises an ear-shaped filter coefficient and a recruitment coefficient; and
the server is specifically configured to: perform ear-shaped migration processing on the first audio data based on the ear-shaped filter coefficient, to obtain first intermediate audio data; perform cochlear conversion processing on the first intermediate audio data, to obtain second intermediate audio data; perform hair cell filtering processing on the second intermediate audio data based on the hair cell filter coefficient, to obtain third intermediate audio data; perform spectral smearing processing on the third intermediate audio data, to obtain fourth intermediate audio data; perform loudness recruitment processing on the fourth intermediate audio data based on the recruitment coefficient, to obtain fifth intermediate audio data; and perform cochlear reverse conversion processing on the fifth intermediate audio data, to obtain the second audio data.

29. The server according to claim 27 or 28, wherein
before performing hair cell filtering processing on the first audio data based on the hair cell filter coefficient, to obtain the second audio data, the server is further configured to:
when the listening test data further comprises the hearing aid type information, perform hearing aid optimization processing on the first audio data by using a hearing aid algorithm corresponding to the hearing aid type information;
when the listening test data further comprises a first hearing aid algorithm parameter, perform hearing aid optimization processing on the first audio data based on the first hearing aid algorithm parameter; or
when the listening test data further comprises an optimization parameter, search for a second hearing aid algorithm parameter based on the optimization parameter; and perform hearing aid optimization processing on the first audio data based on the second hearing aid algorithm parameter.

30. The server according to any one of claims 27 to 29, wherein
the hearing loss feature information comprises at least one of the following: an ear-shaped feature, hearing loss frequency band information, an intelligibility score, an intelligibility confusion matrix, or age-related decline information.

31. A hearing loss simulation apparatus, wherein the hearing loss simulation
apparatus is configured to:
obtain first audio data;
perform ear-shaped migration processing on the first audio data, to obtain first intermediate audio data;
perform cochlear conversion processing on the first intermediate audio data, to obtain second intermediate audio data;
perform hair cell filtering processing on the second intermediate audio data based on the hair cell filter coefficient, to obtain third intermediate audio data;
perform spectral smearing processing on the third intermediate audio data, to obtain fourth intermediate audio data;
perform loudness recruitment processing on the fourth intermediate audio data, to obtain fifth intermediate audio data; and
perform cochlear reverse conversion processing on the fifth intermediate audio data, to obtain second audio data.

32. A computer-readable storage medium, wherein the computer-readable storage
medium stores a computer program, and when the computer program is run on a computer or a processor, the computer or the processor is enabled to perform the steps according to any one of claims 22 to 30.

33. A computer program product, wherein the computer program product comprises
computer instructions, and when the computer instructions are executed by a computer or a processor, the steps according to any one of claims 22 to 30 are performed.
